# EUROPEAN PATENT APPLICATION

(11) **EP 4 325 506 A1**
(43) Date of publication of application: **21.02.2024**
(21) Application number: 23191896.2
(22) Date of filing: 17.08.2023
(51) Int. Cl.: G16B 45/00

(54) **INFORMATION PROCESSING METHOD, INFORMATION PROCESSING APPARATUS, AND TEST SYSTEM**

(30) Priority: 18.08.2022 JP 2022130815
(71) Applicant: SYSMEX CORPORATION, Kobe-shi Hyogo 651-0073 (JP)
(72) Inventor: Karino, Takashi, Kobe-shi, Hyogo, 651-0073 (JP); Hirayama, Hideki, Kobe-shi, Hyogo, 651-0073 (JP)
(74) Representative: Hoffmann Eitle

(57) **Abstract**

Disclosed is an information processing method to be executed by a computer, for analysis performed by a flow cytometer capable of acquiring a test result through a test using an antibody reagent, the information processing method including: acquiring a first test result obtained through a first test performed by the flow cytometer with respect to a first specimen of a subject; acquiring a second test result obtained through a second test different from the first test with respect to a second specimen of the subject; and displaying the first test result and the second test result in association with each other.

## Description

### CROSS REFERENCE TO RELATED APPLICATIONS

This application claims priority from prior Japanese Patent Application No. 2022-130815, filed on August 18, 2022, entitled "INFORMATION PROCESSING METHOD, INFORMATION PROCESSING APPARATUS, AND TEST SYSTEM", the entire content of which is incorporated herein by reference.

### FIELD OF THE INVENTION

The present invention relates to an information processing method, an information processing apparatus, and a test system.

### BACKGROUND OF THE INVENTION

To date, analysis of samples using a flow cytometer has been performed. For example, when, through analysis of blood samples performed by a blood analyzer, a high white blood cell count has been obtained or a possibility of leukemia has been detected, such samples may be analyzed by a flow cytometer by using an antibody reagent.

Japanese Patent No. 5636381 describes an analysis system including: a sample dispenser that divides a blood sample of a subject into a first aliquot and a second aliquot; a flow cytometer that analyzes the first aliquot; and a blood analyzer that analyzes the second aliquot. In this analysis system, analysis results of the first aliquot and the second aliquot divided from the same blood sample are displayed on a display of a user interface.

In a test by a flow cytometer, in order to detect or determine a disease, various types of antigens may be measured. When various types of antigens are measured by the flow cytometer, a clinical technologist examines whether the reliabilities of the respective measurement results of the various types of antigens are high, with reference to another test result of a specimen of the same subject.

The test by the flow cytometer is often performed in a laboratory different from that where the other tests are performed. However, data linkage between laboratories is not sufficient, the above-mentioned examination takes time, and the tests are not efficiently performed, which have been problems.

In view of these problems, an object of the present invention is to provide an information processing method, an information processing apparatus, and a test system that contribute to increase in the efficiency of tests.

### SUMMARY OF THE INVENTION

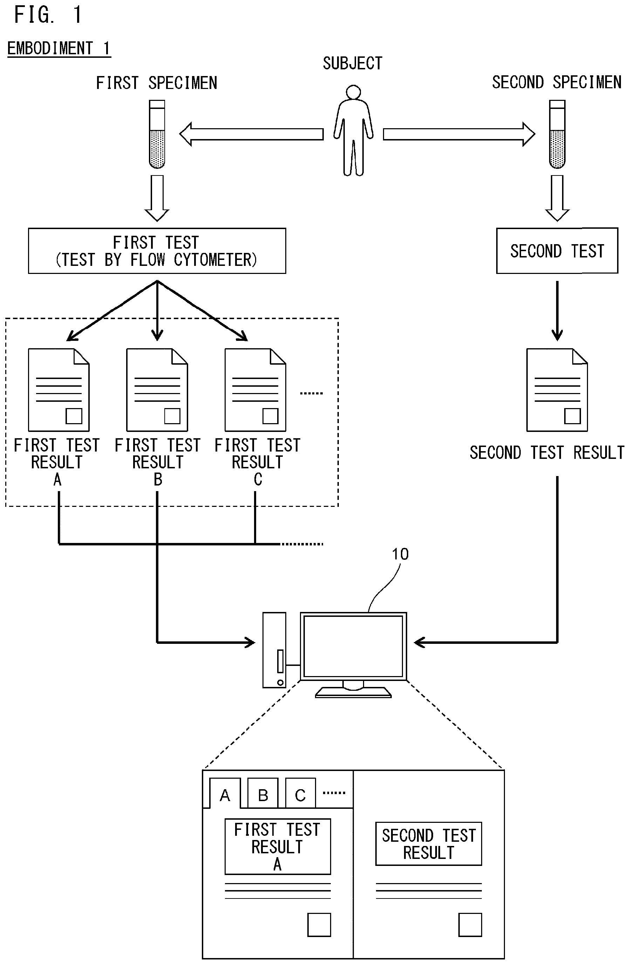

As shown in FIGS. 1, 2, 5, 20, 40, an information processing method of the present invention relates to an information processing method to be executed by a computer (10), for analysis performed by a flow cytometer (21, 21b) capable of acquiring a test result through a test using an antibody reagent. The information processing method of the present invention includes: acquiring (S 1, S 18) a first test result obtained through a first test performed by the flow cytometer (21, 21b) with respect to a first specimen of a subject; acquiring (S 1, S 12) a second test result obtained through a second test different from the first test with respect to a second specimen of the subject; and displaying (S3, S21) the first test result and the second test result in association with each other.

According to the information processing method of the present invention, with respect to specimens of an identical subject, a laboratory technician can grasp the first test result obtained through the first test performed by the flow cytometer and the second test result obtained through the second test different from the first test, in association with each other. Therefore, the laboratory technician can increase the efficiency of the test according to the flow cytometer in consideration of the second test result obtained through the second test.

As shown in FIGS. 1, 2, 3, 40, an information processing apparatus (10) of the present invention relates to an information processing apparatus that is used in analysis performed by a flow cytometer (21, 21b) capable of acquiring a test result through a test using an antibody reagent. The information processing apparatus (10) of the present invention includes a controller (101) and a display unit (103). The controller (101) is configured to acquire a first test result obtained through a first test performed by the flow cytometer (21, 21b) with respect to a first specimen of a subject, acquire a second test result obtained through a second test different from the first test with respect to a second specimen of the subject, and cause the display unit (103) to display the first test result and the second test result in association with each other.

According to the information processing apparatus of the present invention, with respect to specimens of an identical subject, a laboratory technician can grasp, with reference to the display unit, the first test result obtained through the first test performed by the flow cytometer and the second test result obtained through the second test different from the first test, in association with each other. Therefore, the laboratory technician can increase the efficiency of the test according to the flow cytometer in consideration of the second test result obtained through the second test.

As shown in, FIGS. 1, 2, 3, 35, 40, a test system (1) of the present invention includes a flow cytometer (21, 21b) capable of acquiring a test result through a test using an antibody reagent; a test apparatus (22, 22a, 22b) configured to perform a second test different from a first test performed by the flow cytometer (21, 21b); and an information processing apparatus (10) connected to the flow cytometer (21, 21b) and the test apparatus (22, 22a, 22b) through a network (30). The information processing apparatus (10) includes a controller (101) and a display unit (103). The controller (101) is configured to acquire a first test result obtained through a first test performed by the flow cytometer (21, 21b) with respect to a first specimen of a subject, acquire a second test result obtained through a second test different from the first test with respect to a second specimen of the subject, and cause the display unit (103) to display the first test result and the second test result in association with each other.

According to the test system of the present invention, with respect to specimens of an identical subject, a laboratory technician can grasp, with reference to the display unit, the first test result obtained through the first test performed by the flow cytometer and the second test result obtained through the second test different from the first test, in association with each other. Therefore, the laboratory technician can increase the efficiency of the test according to the flow cytometer in consideration of the second test result obtained through the second test.

According to the present invention, efficiency of tests can be increased.

### BRIEF DESCRIPTION OF THE DRAWINGS

FIG. 1 schematically shows an outline of an information processing method according to Embodiment 1;
FIG. 2 is a block diagram showing a configuration of a test system according to Embodiment 1;
FIG. 3 is a block diagram showing a configuration of an information processing apparatus according to Embodiment 1;
FIG. 4 schematically shows items stored in a database, according to Embodiment 1;
FIG. 5 is a flowchart showing a process performed by an information processing apparatus, according to Embodiment 1;
FIG. 6 illustrates Display Examples 1, 2 of a first test result and a second test result displayed on a display unit of the information processing apparatus, according to Embodiment 1;
FIG. 7 illustrates Display Examples 3, 4 of the first test result and the second test result displayed on the display unit of the information processing apparatus, according to Embodiment 1;
FIG. 8 illustrates Display Example 5 of the first test result and the second test result displayed on the display unit of the information processing apparatus, according to Embodiment 1;
FIG. 9 illustrates Display Example 6 of the first test result and the second test result displayed on the display unit of the information processing apparatus, according to Embodiment 1;
FIG. 10 schematically shows items stored in a database, according to Embodiment 2;
FIG. 11 is a table showing examples of a single antibody and examples of a cocktail antibody, according to Embodiment 2;
FIG. 12 is a table showing examples of a panel, according to Embodiment 2;
FIG. 13 schematically shows a configuration of a panel editing screen displayed on the display unit of the information processing apparatus, according to Embodiment 2;
FIG. 14 schematically shows a configuration of an inquiry screen for the second test result displayed on the display unit of the information processing apparatus, according to Embodiment 2;
FIG. 15 schematically shows a configuration of a specimen list display screen displayed on the display unit of the information processing apparatus, according to Embodiment 2;
FIG. 16 schematically shows a configuration of a test order registration screen for the first test displayed on the display unit of the information processing apparatus, according to Embodiment 2;
FIG. 17 schematically shows another configuration of the test order registration screen for the first test displayed on the display unit of the information processing apparatus, according to Embodiment 2;
FIG. 18 schematically shows a configuration of a test order display screen for the first test displayed on the display unit of the information processing apparatus, according to Embodiment 2;
FIG. 19 schematically shows an example of a data structure for storing the first test result, according to Embodiment 2;
FIG. 20 is a flowchart showing a process performed by the information processing apparatus, according to Embodiment 2;
FIG. 21 is a flowchart showing a process performed by the information processing apparatus, according to Modification 1 of Embodiment 2;
FIG. 22 schematically shows a state where a display screen for the second test result is displayed together with the test order registration screen for the first test, on the display unit of the information processing apparatus, according to Modification 1 of Embodiment 2;
FIG. 23 is a flowchart showing a process performed by the information processing apparatus, according to Modification 2 of Embodiment 2;
FIG. 24 is a table showing an example of rules for setting the first test, according to Modification 2 of Embodiment 2;
FIG. 25 is a block diagram showing a configuration of the test system according to Modification 3 of Embodiment 2;
FIG. 26 is a flowchart showing a process performed by the information processing apparatus, according to Modification 3 of Embodiment 2;
FIG. 27 is a flowchart showing another process performed by the information processing apparatus, according to Modification 3 of Embodiment 2;
FIG. 28 is a block diagram showing a configuration of a first test apparatus according to Embodiment 3;
FIG. 29 schematically shows a configuration of a detector according to Embodiment 3;
FIG. 30 is a block diagram showing a configuration of a second test apparatus according to Embodiment 3;
FIG. 31 schematically shows configurations of an optical detector and an electric-resistance-type detector according to Embodiment 3;
FIG. 32 schematically shows a configuration of a test result screen displayed on the display unit of the information processing apparatus according to Embodiment 3;
FIG. 33 is a block diagram showing a configuration of the second test apparatus according to Modification 1 of Embodiment 3;
FIG. 34 schematically shows a configuration of the test result screen displayed on the display unit of the information processing apparatus, according to Modification 1 of Embodiment 3;
FIG. 35 is a block diagram showing a configuration of the test system according to Modification 2 of Embodiment 3;
FIG. 36 schematically shows a configuration of the test result screen displayed on the display unit of the information processing apparatus, according to Modification 2 of Embodiment 3;
FIG. 37 is a block diagram showing a configuration of the second test apparatus according to Modification 3 of Embodiment 3;
FIG. 38 schematically shows a procedure of sample preparation performed by the second test apparatus, according to Modification 3 of Embodiment 3;
FIG. 39 schematically shows a configuration of the test result screen displayed on the display unit of the information processing apparatus, according to Modification 3 of Embodiment 3;
FIG. 40 is a block diagram showing a configuration of the test system according to Embodiment 4;
FIG. 41 is a block diagram showing a configuration of a specimen pretreatment device according to Embodiment 4;
FIG. 42 is a plan view schematically showing a configuration of the specimen pretreatment device according to Embodiment 4;
FIG. 43 schematically shows a configuration of the test order registration screen for the first test displayed on the display unit of the information processing apparatus, according to Embodiment 4;
FIG. 44 schematically shows a configuration of the test order registration screen for the first test displayed on the display unit of the information processing apparatus, according to Embodiment 4;
FIG. 45 schematically shows another configuration of the test order registration screen for the first test displayed on the display unit of the information processing apparatus, according to Embodiment 4;
FIG. 46 schematically shows a configuration of the test order display screen for the first test displayed on the display unit of the information processing apparatus, according to Embodiment 4;
FIG. 47 is a flowchart showing a process performed by the information processing apparatus, according to Embodiment 4;
FIG. 48 is a block diagram showing a configuration of the first test apparatus according to a modification of Embodiment 4; and
FIG. 49 is a plan view schematically showing a configuration of the first test apparatus according to a modification of Embodiment 4.

### DETAILED DESCRIPTION

### <Embodiment 1>

FIG. 1 schematically shows an outline of an information processing method according to Embodiment 1.

A first specimen and a second specimen are collected from a subject. Each of the first specimen and the second specimen is a blood specimen, and in Embodiment 1, is a whole blood specimen. The first specimen and the second specimen may be two specimens collected at an identical timing from an identical subject, or may be two specimens collected at different timings (e.g., different days and times) from an identical subject. The first specimen is subjected to a first test, and the second specimen is subjected to a second test different from the first test.

The first test is analysis regarding an antigen present at least at one of a cell surface and inside of a cell. In the first test, a flow cytometer is used. The flow cytometer of Embodiment 1 is capable of acquiring, according to a flow cytometry method, a plurality of first test results by performing the first test using a plurality of types of antibody reagents with respect to the first specimen of the subject. In the first test, an antigen at each cell in the first specimen is fluorescence-labeled by an antibody reagent, analysis is performed on the basis of fluorescence generated from a fluorescent dye bound to the antigen at the cell, and a first test result is acquired. A plurality of first test results include information on an antigen of a type corresponding to each of a plurality of types of antibody reagents. In the example shown in FIG. 1, as a plurality of first test results, first test results (A, B, C, ...) are acquired. Each first test result is an analysis result based on data obtained through measurement in the first test, but may include both of data and an analysis result.

The second test includes a hematology test. In the hematology test, parameter information (scattered light intensity, fluorescence intensity, electric resistance, change in permittivity based on high-frequency current, electric conductivity, coagulation time, concentration, etc.) indicating the features of each cell itself and the features of components in blood (e.g., size, volume, granule density, stainability, absorbance, etc.) is acquired and analyzed.

In the second test of the present embodiment, a blood cell counter is used, for example. The blood cell counter performs a blood cell test by an optical detector or an electric-resistance-type detector with respect to the second specimen of the subject, acquires parameter information (scattered light intensity, fluorescence intensity, electric resistance, etc.) indicating the features (e.g., size, volume, granule density, stainability, etc.) of each cell itself in the second specimen, and acquires a second test result such as a blood cell count, on the basis of the acquired parameter information. In the example shown in FIG. 1, as the second test result, a second test result regarding a plurality of types of blood cells included in the second specimen is acquired. The second test result is an analysis result based on data obtained through measurement in the second test, but may include both of data and an analysis result obtained through the second test.

In the second test, not limited to using only a blood cell counter, one or more test apparatuses out of apparatuses such as a blood cell counter, a blood cell image analyzer, a genetic test apparatus, and a blood coagulation test apparatus may be used. When a genetic test apparatus is used, the second specimen only needs to include cells of a subject, and may be a specimen other than blood specimen. When a blood coagulation test apparatus is used, the second specimen is plasma of a subject.

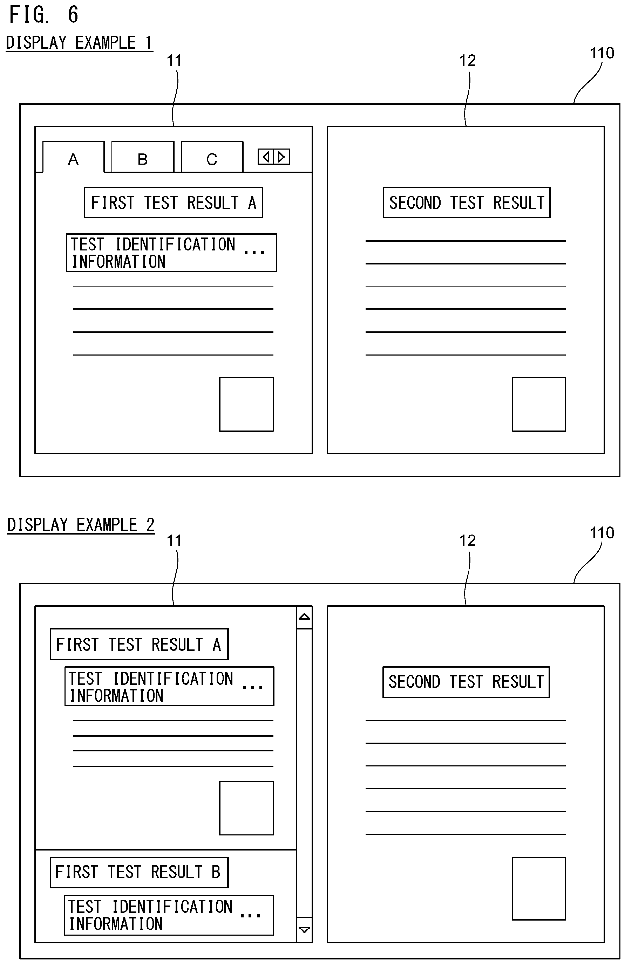

An information processing apparatus 10 is used in analysis performed by a flow cytometer that performs the first test. The information processing apparatus 10 acquires the first test result obtained through the first test and the second test result obtained through the second test, and causes a display unit to display the first test result and the second test result in association with each other. Displaying the first test result and the second test result in association with each other includes, for example, displaying the first test result and the second test result corresponding to a common subject so as to be arranged side by side on one, two, or more screens so as to be contrastable with each other; displaying, by switching through operation of a user interface (e.g., tab) displayed on a screen, the first test result and the second test result so as to be contrastable with each other; and the like. Displaying the first test result and the second test result so as to be contrastable with each other will be described later with reference to FIGS. 6 to 9.

Displaying the first test result and the second test result in association with each other is not limited to displaying the first test result and the second test result so as to be contrastable with each other. For example, the first test result and the second test result are associated with each other as data, and in accordance with an instruction by an operator, one of the first test result and the second test result may be displayed on a display unit.

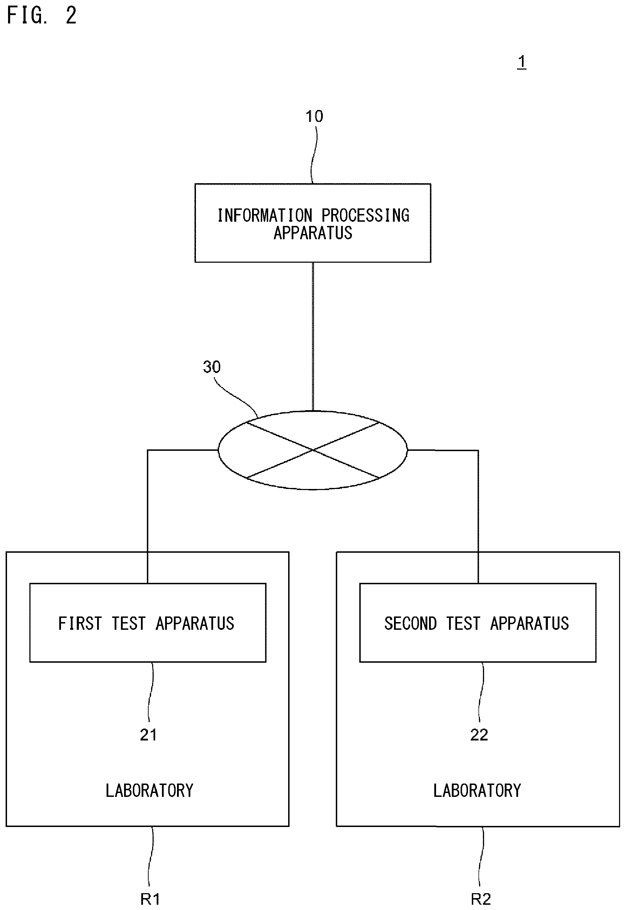

FIG. 2 is a block diagram showing a configuration of a test system 1.

The test system 1 includes the information processing apparatus 10, a first test apparatus 21, and a second test apparatus 22.

The first test apparatus 21 is a flow cytometer capable of acquiring a plurality of test results through tests using a plurality of types of antibody reagents. The first test apparatus 21 performs the first test. The second test apparatus 22 is an apparatus that performs the second test different from the first test. A laboratory R1 where the first test apparatus 21 is installed and a laboratory R2 where the second test apparatus 22 is installed are different from each other. That is, the first test is performed in a laboratory different from that where the second test is performed.

The first test apparatus 21 and the second test apparatus 22 may be installed in the same laboratory. That is, the first test and the second test may be performed in the same laboratory. In this case, the first test apparatus 21 and the second test apparatus 22 are installed at different places in the same laboratory.

The information processing apparatus 10 is an apparatus connected to the first test apparatus 21 and the second test apparatus 22 through a network 30. The network 30 is implemented by the Internet or an intranet. The network 30 may be implemented by the Internet and an intranet.

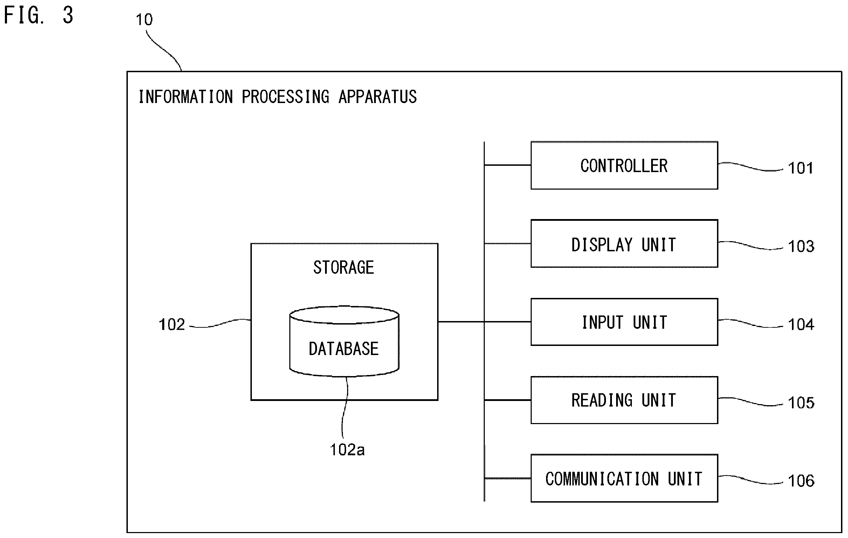

FIG. 3 is a block diagram showing a configuration of the information processing apparatus 10.

The information processing apparatus 10 includes a controller 101, a storage 102, a display unit 103, an input unit 104, a reading unit 105, and a communication unit 106. An operator who uses the information processing apparatus 10 is a laboratory technician, for example.

The controller 101 is implemented by a CPU, for example. The controller 101 is connected to each component in the information processing apparatus 10 through a bus, an interface, or the like. The controller 101 executes a program read out from the storage 102. By executing the program, the controller 101 displays the first test result and the second test result in association with each other, as shown in FIG. 1.

The storage 102 is implemented by an HDD or an SSD, for example. The storage 102 stores therein a database 102a storing the first test result, the second test result, and the like. The storage 102 stores therein a program for displaying the first test result and the second test result, a program for displaying various screens, and the like.

The display unit 103 is implemented by a liquid crystal display, for example. The input unit 104 is implemented by a pointing device and the like including a keyboard, a mouse, and a touch panel, for example. The operator operates the input unit 104, to operate an operation unit such as a button in a screen displayed on the display unit 103. The display unit 103 and the input unit 104 may be integrally formed. For example, the information processing apparatus 10 may include a touch-panel type display as the display unit 103 and the input unit 104.

The reading unit 105 is implemented by a barcode reader, for example. The communication unit 106 is a communication interface based on the Ethernet standard, for example. The controller 101 communicates with the first test apparatus 21 and the second test apparatus 22 through the communication unit 106.

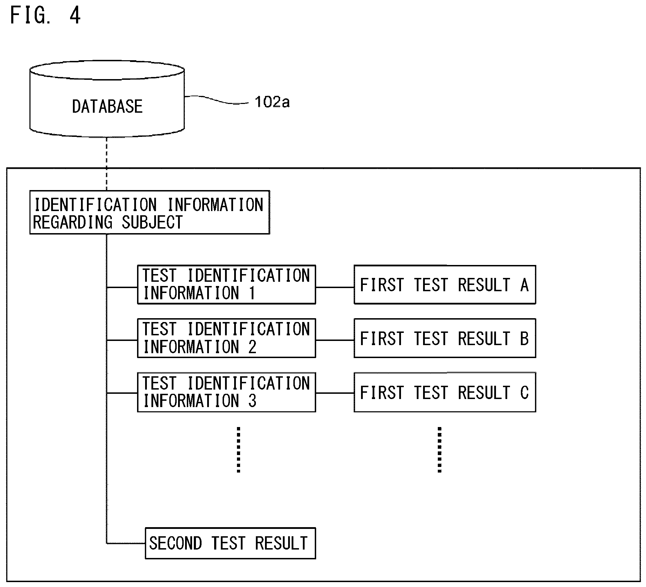

FIG. 4 schematically shows items stored in the database 102a.

The database 102a includes items such as identification information regarding a subject, a plurality of pieces of test identification information, a plurality of first test results, one second test result, and the like. In the database 102a, the plurality of pieces of test identification information (1, 2, 3, ...) are associated with the identification information regarding the subject, and the first test results (A, B, C, ....) are associated with the plurality of pieces of test identification information, respectively. One second test result is associated with the identification information regarding the subject.

As the identification information regarding the subject, for example, at least one of a subject ID capable of identifying the subject and a specimen ID capable of identifying a specimen is included. For example, when the first specimen subjected to the first test and the second specimen subjected to the second test are two specimens collected at an identical timing from an identical subject, the specimen ID provided to the first specimen and the specimen ID provided to the second specimen may be identical to each other. In this case, as the identification information regarding the subject, at least one of the subject ID and the specimen ID can be used. When the first specimen and the second specimen are two specimens collected at different timings from an identical subject, the specimen ID provided to the first specimen and the specimen ID provided to the second specimen may be different from each other. In this case, as information regarding the subject, the subject ID can be used.

The test identification information is information capable of identifying a plurality of tests performed in the first test. The test identification information is stored in a storage in advance in the first test apparatus 21, for example. In this case, upon ending the first test, the first test apparatus 21 transmits the information regarding the subject, the test identification information, and the first test result in association with each other, to the information processing apparatus 10. The test identification information may be set as desired by the operator through the input unit 104. In this case, the controller 101 of the information processing apparatus 10 transmits the inputted test identification information to the first test apparatus 21, and the first test apparatus 21 stores the received test identification information into a storage.

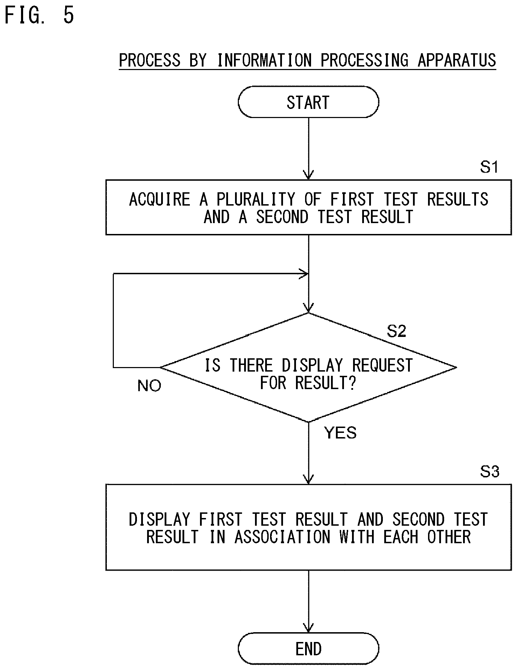

FIG. 5 is a flowchart showing a process performed by the information processing apparatus 10.

In step S1, the controller 101 of the information processing apparatus 10 acquires first test results from the first test apparatus 21 and acquires a second test result from the second test apparatus 22. In step S2, the controller 101 determines whether or not a display request for a result has been inputted from the operator through the input unit 104. When the display request for the result has been inputted, the controller 101 causes, in step S3, the display unit 103 to display each first test result and the second test result in association with each other.

Next, with reference to FIGS. 6 to 9, Display Examples 1 to 6 of the first test result and the second test result displayed on the display unit 103 of the information processing apparatus 10 will be described. In the examples shown in FIGS. 6 to 9, the first test results and the second test result are displayed on the display unit 103 such that each of the plurality of first test results and the second test result are contrastable with each other.

As shown in the upper part of FIG. 6, in Display Example 1, a test result screen 110 including a first region 11 for displaying each of the plurality of first test results and a second region 12 for displaying the second test result is displayed on the display unit 103. In the test result screen 110, the first region 11 and the second region 12 are disposed so as to be arranged in the left-right direction.

The first region 11 is provided with tabs for switching displays of the plurality of first test results having been acquired. By operating a tab, the operator can cause a desired first test result, out of the plurality of first test results, to be displayed in the first region 11. The first test result in the first region 11 includes text information (e.g., characters or numerical values for indicating the count of blood cells having expression of an antigen to be measured) and a graph (e.g., a scattergram or a histogram). In the example shown in the upper part of FIG. 6, a tab corresponding to a first test result A is operated and the first test result A is displayed in the first region 11. In addition, in the first region 11, the test identification information corresponding to the first test result being displayed is displayed.

As shown in the lower part of FIG. 6, in Display Example 2, the layout of the first region 11 is changed when compared with Display Example 1. In Display Example 2, the plurality of first test results are arranged in the vertical direction, and a scroll bar for moving the display in the vertical direction is provided. By operating the scroll bar, the operator can cause a first test result as a target, out of the plurality of first test results, to be displayed in the first region 11.

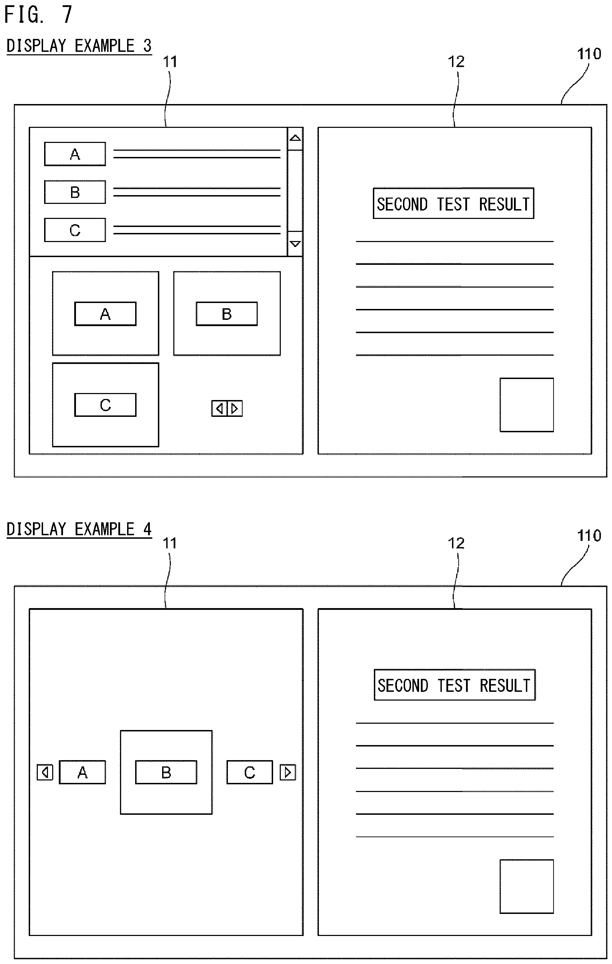

As shown in the upper part of FIG. 7, in Display Example 3, the layout of the first region 11 is changed when compared with Display Example 1. In Display Example 3, in the upper part of the first region 11, the test identification information and the text information regarding each of the plurality of first test results are displayed and a scroll bar for moving these displays in the vertical direction is provided. In the lower part of the first region 11, graphs respectively regarding the plurality of first test results are displayed and an arrow button for switching these displays is provided. When each region displayed in the lower part of the first region 11 is operated, the first test result corresponding to the region may be expanded to the entirety of the first region 11.

As shown in the lower part of FIG. 7, in Display Example 4, the layout of the first region 11 is changed when compared with Display Example 1. In Display Example 4, at the center of the first region 11, a region in which details of a first test result is displayed in a large area is provided, and to the left and the right of this region, regions in which the summaries of first test results are displayed in small areas are provided. At the left end and the right end of the first region 11, buttons for switching the first test result of which the details are to be displayed at the center of the first region 11 is provided. When the region at the center of the first region 11 is operated, the first test result corresponding to this region may be expanded to the entirety of the first region 11.

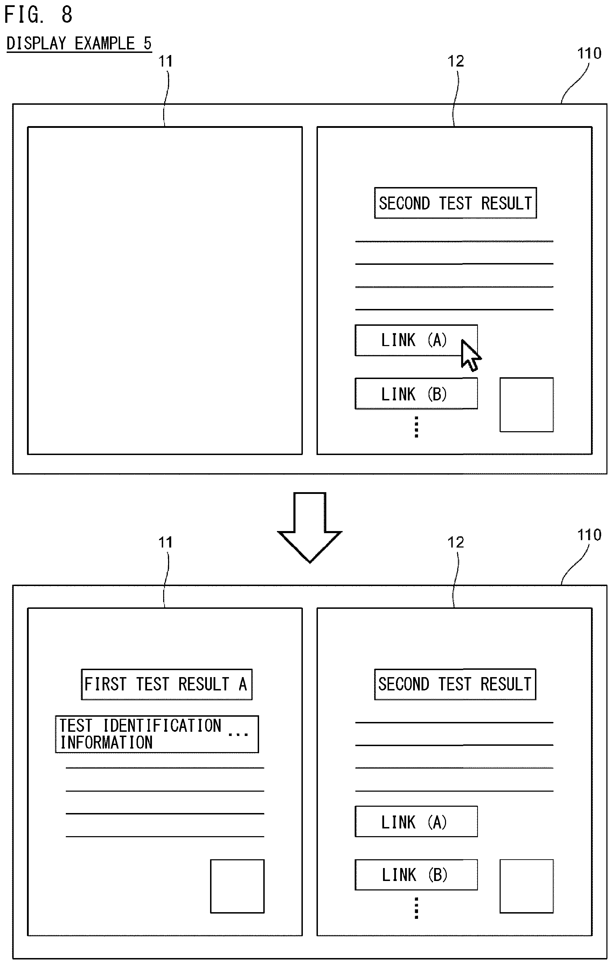

As shown in FIG. 8, in Display Example 5, in the first region 11, one first test result is displayed, and in the second region 12, link buttons each for designating a first test result, out of the plurality of first test results, to be displayed in the first region 11 are provided, when compared with Display Example 1. A plurality of link buttons are provided so as to correspond to the plurality of first test results having been acquired. In Display Example 5, the state shown in the upper part of FIG. 8 is the initial state. In this state, when the operator operates a link button in the second region 12, details of the first test result corresponding to the link button are displayed in the first region 11, as shown in the lower part of FIG. 8.

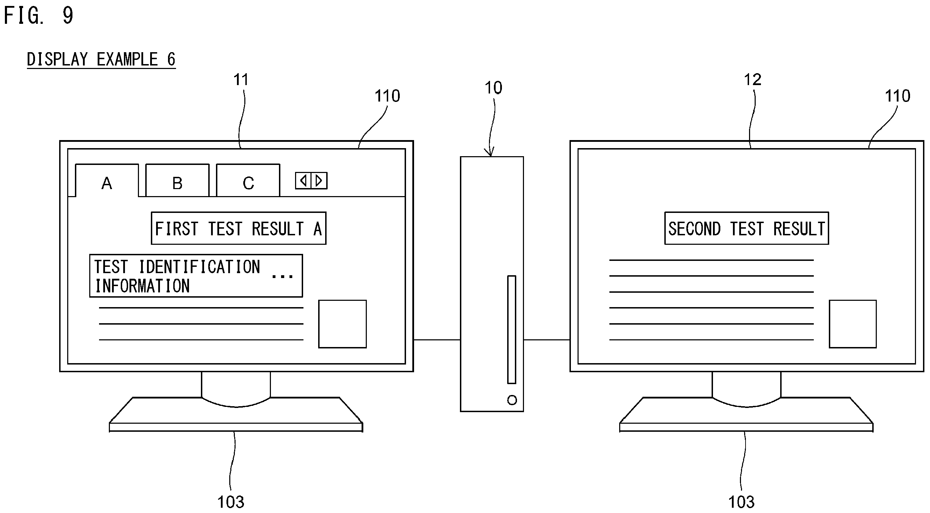

As shown in FIG. 9, in Display Example 6, the information processing apparatus 10 includes two display units 103, and the two display units 103 form a so-called dual display. In Display Example 6, the test result screen 110 is displayed so as to extend across the two display units 103, and the first region 11 is disposed in the test result screen 110 of the display unit 103 on the left side, and the second region 12 is disposed in the test result screen 110 of the display unit 103 on the right side.

Separate test result screens may be displayed in the two display units 103, respectively, and the first region 11 and the second region 12 may be disposed in the test result screens, respectively. The number of the display units 103 may be three or more. In this case, the three or more display units 103 form a so-called multi-display, and the first region 11 and the second region 12 are displayed on the display units 103, as appropriate.

In Display Examples 1 to 6, the test result screen 110 is displayed in a state where the display face of the display unit 103 is laterally long, but the test result screen 110 may be displayed in a state where the display face of the display unit 103 is vertically long. In this case, for example, in the test result screen 110 that is vertically long, the first region 11 and the second region 12 are disposed so as to be arranged in the up-down direction.

### <Effects of information processing method, information processing apparatus, and test system according to Embodiment 1>

The controller 101 of the information processing apparatus 10 acquires the first test result obtained through the first test performed by the first test apparatus 21 (flow cytometer) with respect to the first specimen of a subject, and acquires the second test result obtained through the second test different from the first test with respect to the second specimen of the subject. Then, the controller 101 causes the display unit 103 to display the first test result and the second test result in association with each other. Accordingly, with respect to the specimens of an identical subject, the operator can grasp the first test result obtained through the first test and the second test result obtained through the second test different from the first test in association with each other. Therefore, the operator can increase the efficiency of the test according to the first test apparatus 21 (flow cytometer) in consideration of the second test result obtained through the second test.

In the examples shown in FIGS. 6 to 9, the controller 101 causes the display unit 103 to display the first and second test results such that each of the plurality of first test results and the second test result are contrastable with each other. Accordingly, the operator can evaluate the validity of the first test results in consideration of the second test, and thus, can further increase the efficiency of the test according to the first test apparatus 21 (flow cytometer).

For example, when, with reference to the first test result and the second test result displayed in contrast, the operator has determined that the first test result and the second test result are contradictory with each other, the operator can determine that the validity of the first test result is low. In this case, the operator does not report the first test result to the doctor. Meanwhile, when, with reference to the first test result and the second test result displayed in contrast, the operator has determined that the first test result and the second test result show similar tendencies, the operator can determine that the validity of the first test result is high. In this case, the operator reports the first test result to the doctor. Thus, the operator can evaluate the validity of the test result of the first test apparatus 21 efficiently and in a simple manner in terms of so-called validation, and can take the next action such as reporting to the doctor, on the basis of the evaluation of the validity.

In a case where a predetermined result (e.g., a result indicating that there are many blast cells) has been obtained as the second test result, the operator can determine, with reference to the first test result and the second test result displayed in contrast, whether or not (whether or not the first test result is valid) the first test result includes a result serving as a basis for the predetermined result obtained through the second test result. When the operator determines that the first test result does not include the basis for the predetermined result, the operator creates a test order for the first test again such that the first test result includes a necessary result, and causes the first test apparatus 21 to perform the first test. With reference to the second test result and the first test result acquired again which are displayed in contrast, the operator can determine whether or not (e.g., whether or not the first test result reinforces the basis) the first test result is valid as the basis for the predetermined result obtained through the second test result. In this manner, the operator can evaluate the validity of the first test result efficiently and in a simple manner in terms of an additional test order.

Further, with reference to the first test result and the second test result displayed in association with each other, the operator can evaluate the possibility of a specific disease based on the combination of the first test result and the second test result.

Displaying the first and second test results includes displaying the test result screen 110 including the first region 11 for displaying the first test result and the second region 12 for displaying the second test result. According to this process, as shown in FIGS. 6 to 9, the first test result and the second test result are displayed so as to be arranged side by side in the test result screen 110, and thus, contrasting the first test result and the second test result is facilitated.

As shown in FIG. 6, the first test result and the test identification information are displayed in association with each other in the first region 11. With this configuration, the type of the first test result can be easily and smoothly grasped.

Each of the first specimen and the second specimen is a blood specimen, and the second test includes a hematology test. Therefore, hematology information (blood cell count calculation result, blood cell image test result, etc.) that cannot be obtained through analysis of blood cells by the first test apparatus 21 (flow cytometer) can be confirmed.

As shown in FIG. 2, the first test apparatus 21 (flow cytometer) is installed in the laboratory R1 different from the laboratory R2 where the second test is performed. Thus, even when the first test is performed in a laboratory different from that where the second test is performed, the first test result and the second test result are displayed so as to be contrastable with each other as described above, whereby the first test result can be effectively utilized in consideration of the second test result.

### <Embodiment 2>

In Embodiment 2, when compared with Embodiment 1, the first test result and the second test result are associated with a first specimen ID and a second specimen ID, respectively, and in accordance with operation of the information processing apparatus 10, a test order for the first test is set. Unless otherwise specified, the configuration and the process in Embodiment 2 are similar to those in Embodiment 1.

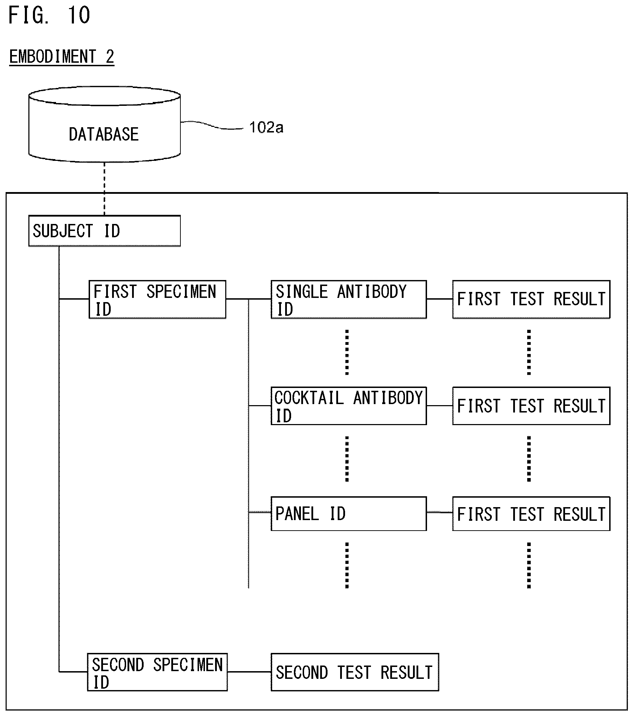

FIG. 10 schematically shows items stored in the database 102a, according to Embodiment 2.

The database 102a in Embodiment 2 includes items such as a subject ID, a first specimen ID, a single antibody ID, a cocktail antibody ID, a panel ID, a first test result, a second specimen ID, and a second test result. In the database 102a, the first specimen ID is associated with the subject ID, and the single antibody ID, the cocktail antibody ID, and the panel ID are associated with the first specimen ID. The respective first test results are associated with the single antibody ID, the cocktail antibody ID, and the panel ID. The second specimen ID is associated with the subject ID, and the second test result is associated with the second specimen ID.

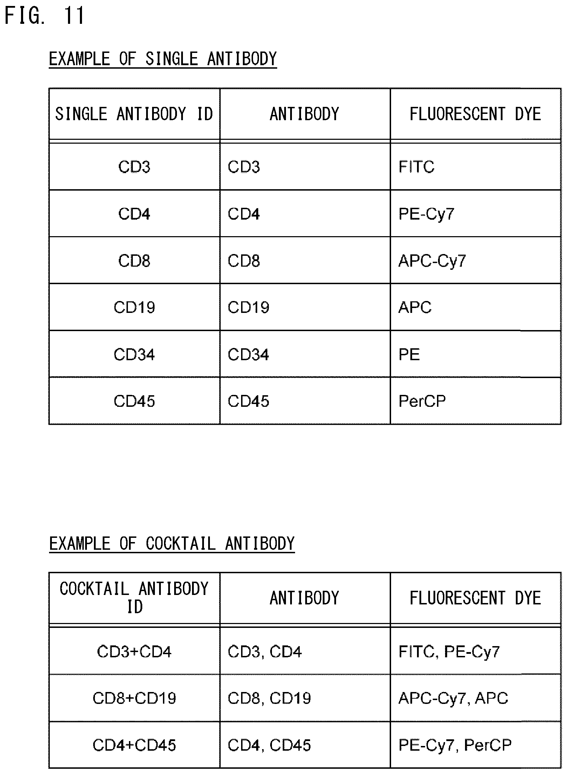

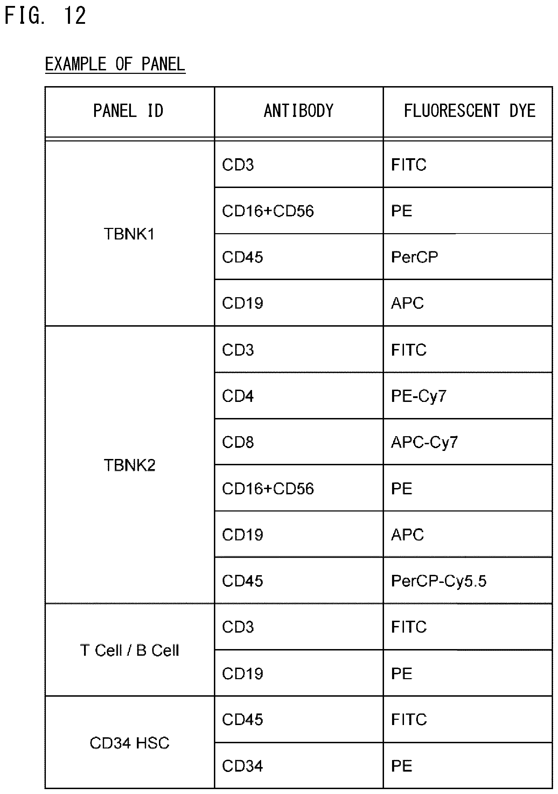

The first specimen ID is an ID for identifying the first specimen to be subjected to the first test, and the second specimen ID is an ID for identifying the second specimen to be subjected to the second test. The single antibody ID, the cocktail antibody ID, and the panel ID correspond to the test identification information in Embodiment 1 shown in FIG. 4, and are information that indicates an antibody reagent to be used in the first test as described later with reference to FIGS. 11, 12.

When a plurality of pieces of the test identification information (the single antibody ID, the cocktail antibody ID, and the panel ID) are set to the subject ID and the first specimen, the first specimen is dispensed from a parent specimen container containing the first specimen into each of child specimen containers prepared for the respective pieces of the test identification information. That is, the first specimen is divided into a plurality of child specimens (aliquots). Then, in each child specimen container, the child specimen (aliquot) and an antibody reagent are mixed together, whereby a measurement sample is prepared. One first test result is obtained from the measurement sample contained in one child specimen container, and thus, when a plurality of pieces of the test identification information are set, a plurality of first test results are obtained. The first test results obtained through the first test are stored in the database 102a in association with the test identification information (the single antibody ID, the cocktail antibody ID, and the panel ID).

The first specimen ID and the second specimen ID may be omitted. In this case, the single antibody ID, the cocktail antibody ID, the panel ID, the first test results, and the second test result are stored in association with the subject ID.

FIG. 11 is a table showing examples of a single antibody and examples of a cocktail antibody.

In the upper part of FIG. 11, six examples of the single antibody are shown, and the single antibody IDs of the respective single antibodies are "CD3", "CD4", "CD8", "CD 19", "CD34", and "CD45". One antibody and a fluorescent dye that binds to this antibody are associated with a single antibody ID. The antibody and the fluorescent dye (i.e., one row in the table) corresponding to one single antibody ID form one antibody reagent, which is also referred to as a "single antibody" in the present embodiment. In a plurality of single antibodies, the antibodies included in the respective single antibodies are different from each other. The single antibody ID only needs to be capable of identifying a single antibody, and may be a name or a number provided to the single antibody, for example.

In the lower part of FIG. 11, three examples of the cocktail antibody are shown, and the cocktail antibody IDs of the respective cocktail antibodies are "CD3+CD4", "CD8+CD19", and "CD4+CD45". Two or more antibodies and fluorescent dyes that respectively bind to these antibodies are associated with a cocktail antibody ID. The plurality of antibodies and the plurality of fluorescent dyes (i.e., one row in the table) corresponding to one cocktail antibody ID form one antibody reagent, which is also referred to as a "cocktail antibody" in the present embodiment. In a plurality of cocktail antibodies, combinations of a plurality of antibodies included in the respective cocktail antibodies are different from each other. The cocktail antibody ID only needs to be capable of identifying a cocktail antibody, and may be a name or a number provided to the cocktail antibody, for example.

FIG. 12 is a table showing examples of a panel.

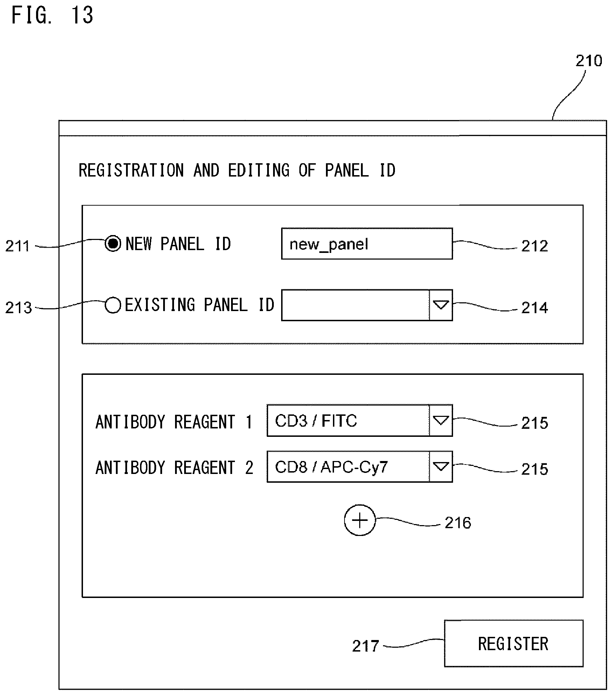

In FIG. 12, four examples of the panel are shown, and the panel IDs of the respective panels are "TBNK1", "TBNK2", "T Cell/B Cell", and "CD34 HSC". A plurality of antibody reagents (i.e., one row in the table) formed by one or more antibodies and one or more fluorescent dyes are associated with a panel ID. A plurality of antibody reagents grouped by the panel ID are also referred to as a "panel" in the present embodiment. In a plurality of panels, combinations of a plurality of antibodies included in the respective panels are different from each other. The panel ID only needs to be capable of identifying a panel, and may be a name or a number provided to the panel, for example. The plurality of antibody reagents grouped by the panel ID can be edited by using a panel editing screen 210 described later with reference to FIG. 13.

As shown in FIGS. 11, 12, one row of "antibody" and "fluorescent dye" corresponds to one antibody reagent, and each of a plurality of types of antibody reagents has a different antibody or a different combination of antibodies. The plurality of antibody reagents are categorized for each container (a child specimen container into which a child specimen has been dispensed from the first specimen) for preparing a measurement sample for the first test. One child specimen container corresponds to one piece of the test identification information (the single antibody ID, the cocktail antibody ID, and the panel ID), and one first test result is obtained from a measurement sample prepared in one child specimen container.

The test identification information (the single antibody ID, the cocktail antibody ID, and the panel ID) in FIGS. 11, 12 is information indicating an antibody reagent to be used in the first test, that is, information capable of specifying an antibody reagent. However, not limited thereto, the test identification information may be, instead of information indicating an antibody reagent, information (e.g., "test 1", "test 2", etc.) capable of simply identifying the test identification information with each other.

The information on the antibody reagent (a combination corresponding to one row of "antibody" and "fluorescent dye") as shown in FIGS. 11, 12 is stored in the storage 102 of the information processing apparatus 10 and a storage of the first test apparatus 21. The single antibody ID, the cocktail antibody ID, and the panel ID as shown in FIGS. 11, 12 and the antibody reagent associated with these IDs are stored in the storage 102 of the information processing apparatus 10 and a storage of the first test apparatus 21.

FIG. 13 schematically shows a configuration of the panel editing screen 210 displayed on the display unit 103 of the information processing apparatus 10.

When registering a new panel ID, the operator selects a radio button 211 and inputs a new panel ID into a text box 212. When editing an existing panel ID, the operator selects a radio button 213 and selects an existing ID from a pulldown menu 214. When selecting a plurality of antibody reagents after selecting one antibody reagent from one pulldown menu 215, the operator operates an addition button 216 to cause the pulldown menu 215 to be additionally displayed.

When a registration button 217 has been operated by the operator, the controller 101 of the information processing apparatus 10 stores, into the storage 102, the information on the panel ID and the combination of antibody reagents set on the panel editing screen 210. In this manner, the operator can set, as desired, the types of antibody reagents associated with a panel ID, through the panel editing screen 210.

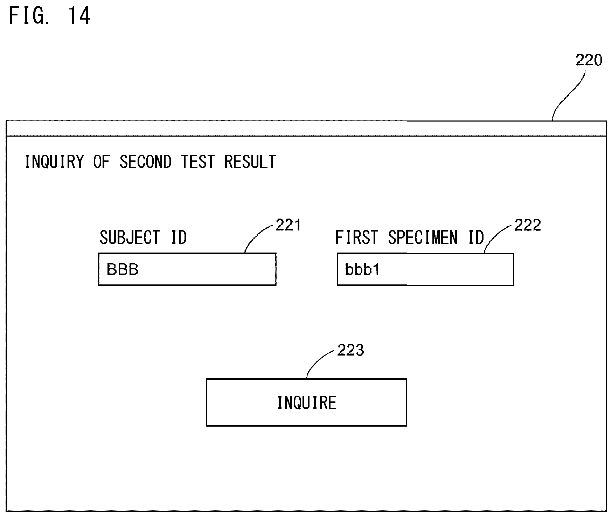

FIG. 14 schematically shows a configuration of an inquiry screen 220 for the second test result displayed on the display unit 103 of the information processing apparatus 10.

The operator inputs a subject ID and a first specimen ID corresponding to the first specimen, into text boxes 221, 222. In the example shown in FIG. 14, "BBB" is inputted as the subject ID, and "bbb1" is inputted as the first specimen ID.

When a label on which a barcode indicating the subject ID and a barcode indicating the first specimen ID are printed is attached to a container containing the first specimen, the operator reads the barcodes by using the reading unit 105 (see FIG. 3), thereby inputting the subject ID and the first specimen ID to the text boxes 221, 222. Other than this, the operator may acquire the subject ID and the first specimen ID from a print and manually input them through the input unit 104.

When an inquiry button 223 has been operated by the operator, the controller 101 of the information processing apparatus 10 transmits an inquiry to the second test apparatus 22 on the basis of the inputted subject ID and first specimen ID, and acquires a second specimen ID and a second test result from the second test apparatus 22. Then, the controller 101 stores, into the database 102a, the second specimen ID and the second test result in association with the subject ID and the first specimen ID.

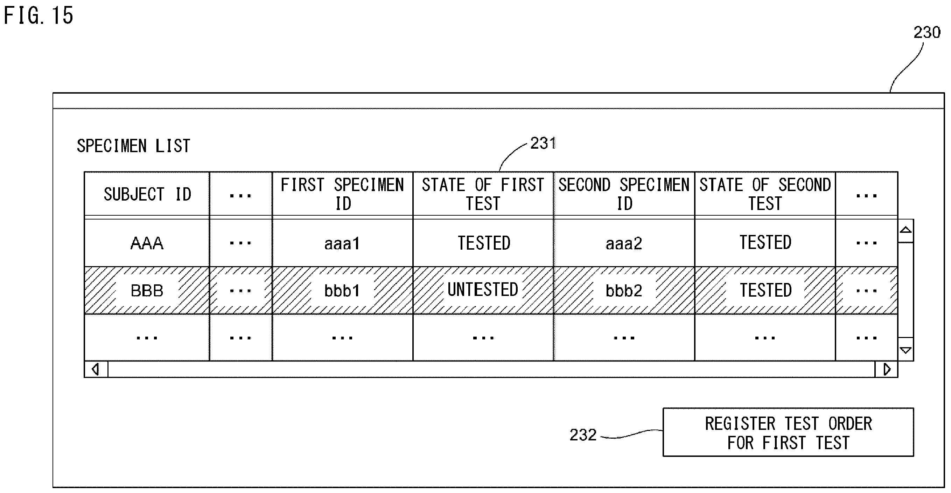

FIG. 15 schematically shows a configuration of a specimen list display screen 230 displayed on the display unit 103 of the information processing apparatus 10.

In a list display region 231, items such as a subject ID, a first specimen ID, a state of the first test, a second specimen ID, and a state of the second test are displayed on the basis of the database 102a. The state of the first test becomes "tested" when the first test result corresponding to the first specimen is stored in the database 102a, and is "untested" when no first test result corresponding to the first specimen is stored in the database 102a. Similarly, the state of the second test becomes "tested" when the second test result corresponding to the second specimen is stored in the database 102a, and is "untested" when no second test result corresponding to the second specimen is stored in the database 102a.

When an inquiry is performed in a state where "BBB" is inputted as the subject ID and "bbb1" is inputted as the first specimen ID in FIG. 14, normally, the first test is not executed and the second test is already executed. Therefore, in this case, as shown in the second row in the list display region 231 in FIG. 15, the state of the first test is "untested", and the state of the second test becomes "tested".

When the operator is to register a test order for the first test, the operator selects a row as a target in the list display region 231, and operates a registration button 232. Accordingly, a test order registration screen 240 for the first test corresponding to the selected subject ID and first specimen ID is displayed.

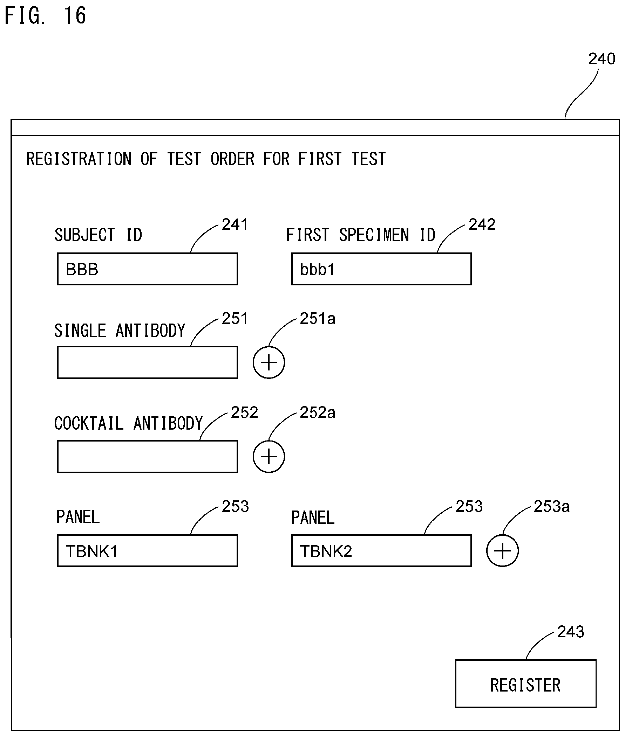

FIG. 16 schematically shows a configuration of the test order registration screen 240 for the first test displayed on the display unit 103 of the information processing apparatus 10.

With respect to a subject and a first specimen for which a test order for the first test is to be registered, the operator inputs the subject ID and the first specimen ID into text boxes 241, 242. In the example shown in FIG. 16, since the registration button 232 has been operated in a state where the second row has been selected in FIG. 15, "BBB" is inputted in advance as the subject ID in the text box 241, and "bbb1" is inputted in advance as the first specimen ID in the text box 242.

The operator inputs the content of the first test into a text box 251, 252, 253. A single antibody ID as shown in the upper part of FIG. 11 is inputted in the text box 251, a cocktail antibody ID as shown in the lower part of FIG. 11 is inputted in the text box 252, and a panel ID as shown in FIG. 12 is inputted in the text box 253. When addition buttons 251a, 252a, 253a are operated, additional text boxes 251, 252, 253 are further displayed on the right side of the text boxes 251, 252, 253 being displayed. Accordingly, input of two or more single antibodies, input of two or more cocktail antibodies, and input of two or more panels are enabled.

In the example shown in FIG. 16, a state where the text box 253 is added through operation of the addition button 253a is shown, and "TBNK1" and "TBNK2" are inputted as the panel IDs in the two text boxes 253.

When a registration button 243 is operated by the operator, the controller 101 of the information processing apparatus 10 stores the test order for the first test into the storage 102.

When the registration button 232 is operated while no row in the list display region 231 is selected in FIG. 15, the text boxes 241, 242 are in a state of being blank. In this case, the operator inputs a subject ID and a first specimen ID by using the input unit 104 or the reading unit 105. Then, when the registration button 243 is operated, the controller 101 stores the test order for the first test into the storage 102, and similar to the process described with reference to FIG. 14, the controller 101 acquires a second specimen ID and a second test result on the basis of the subject ID and the first specimen ID inputted in the text boxes 241, 242 from the second test apparatus 22. The controller 101 stores, into the database 102a, the second specimen ID and the second test result in association with the subject ID and the first specimen ID.

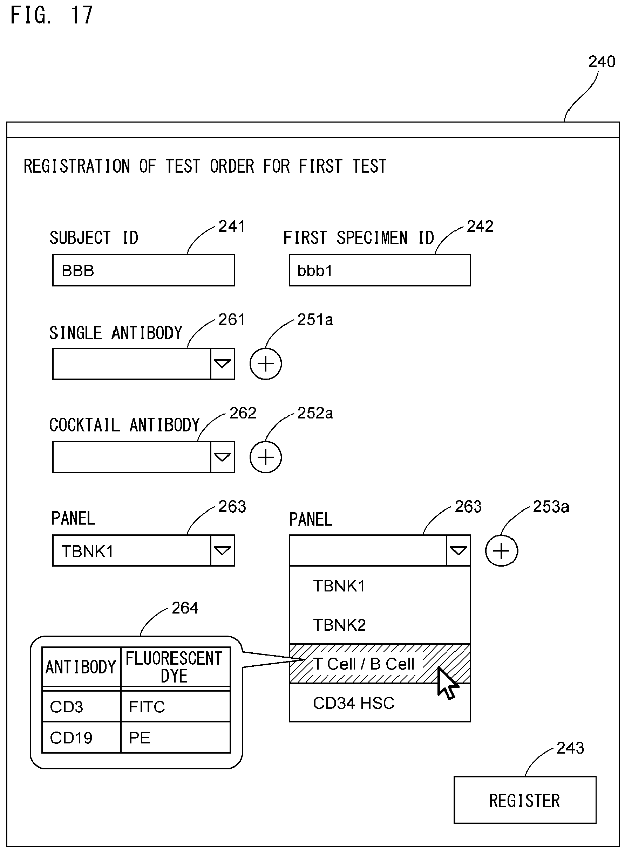

The test order registration screen 240 for the first test shown in FIG. 16 may be configured as shown in FIG. 17.

FIG. 17 schematically shows another configuration of the test order registration screen 240 for the first test displayed on the display unit 103 of the information processing apparatus 10.

In FIG. 17, when compared with FIG. 16, pulldown menus 261, 262, 263 are respectively added in place of the text boxes 251, 252, 253.

The operator operates the pulldown menu 261, 262, 263 to cause the content of the first test to be displayed in the pulldown menu 261, 262, 263. As described above, information on the single antibodies, the cocktail antibodies, and the panels as shown in FIGS. 11, 12 is stored in the storage 102 of the information processing apparatus 10. When the pulldown menus 261, 262, 263 are operated, lists of single antibody IDs, cocktail antibody IDs, and panel IDs stored in the storage 102 are displayed, respectively. The operator selects one item from the list displayed in each pulldown menu, to input a single antibody ID, a cocktail antibody ID, or a panel ID.

When the operator places a selection cursor over each item in a list displayed in the pulldown menu 261, 262, 263, detailed information 264 of the item over which the selection cursor is placed is displayed in the vicinity of the selection cursor. The detailed information 264 is information indicating the antibody reagent corresponding to the single antibodies, the cocktail antibodies, and the panels shown in FIGS. 11, 12. The information indicating the antibody reagent of the detailed information 264 includes an antibody name and a fluorescent dye name. In the example shown in FIG. 17, in the pulldown menu 263, the selection cursor is placed over the item of which the panel ID is "T Cell/B Cell", and information indicating the antibody reagents corresponding to "T Cell/B Cell" is displayed in the detailed information 264.

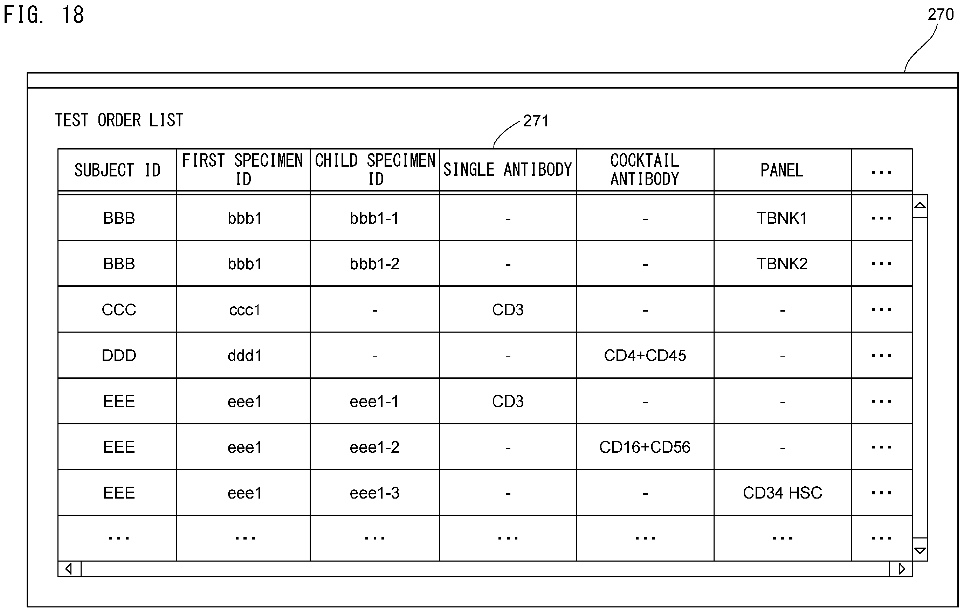

FIG. 18 schematically shows a configuration of a test order display screen 270 for the first test displayed on the display unit 103 of the information processing apparatus 10.

In a list display region 271, items such as a subject ID, a first specimen ID, a child specimen ID, a single antibody ID, a cocktail antibody ID, and a panel ID are displayed on the basis of a test order for the first test registered through the test order registration screen 240.

In the first and second rows in the list display region 271, the test order for the first test registered by the registration button 243 being operated in the state shown in FIG. 16 is shown as an example. In this case, since TBNK1 and TBNK2 are set as the panel IDs in FIG. 16, two test orders are registered so as to correspond to an identical subject ID and an identical first specimen ID, as shown in the first and second rows in FIG. 18. When a plurality of child specimens are made with respect to a first specimen which is a parent specimen, child specimen IDs may be automatically set. In the example shown in the first and second rows in FIG. 18, from a first specimen of which the first specimen ID is "bbb1", two child specimens of which the child specimen IDs are "bbb1-1" and "bbb1-2" are made, and measurement samples are respectively prepared in two containers into which the two child specimens are respectively dispensed.

In the third row in the list display region 271, a test order in which a single antibody is registered is shown as an example. In the fourth row in the list display region 271, a test order in which a cocktail antibody is registered is shown as an example. In the fifth to seventh rows in the list display region 271, test orders in which all of a single antibody, a cocktail antibody, and a panel are registered are shown as an example. In the example shown in the fifth to seventh rows in FIG. 18, child specimen IDs respectively corresponding to a single antibody ID, a cocktail antibody ID, and a panel ID are set, and measurement samples are respectively prepared in three containers into which the three child specimens are respectively dispensed.

After a test order for the first test has been registered, the controller 101 of the information processing apparatus 10 transmits the test order for the first test to the first test apparatus 21. The first test apparatus 21 performs the first test on the basis of the test order for the first test. The controller 101 of the information processing apparatus 10 acquires a first test result obtained through the first test, from the first test apparatus 21, and stores the first test result into the database 102a.

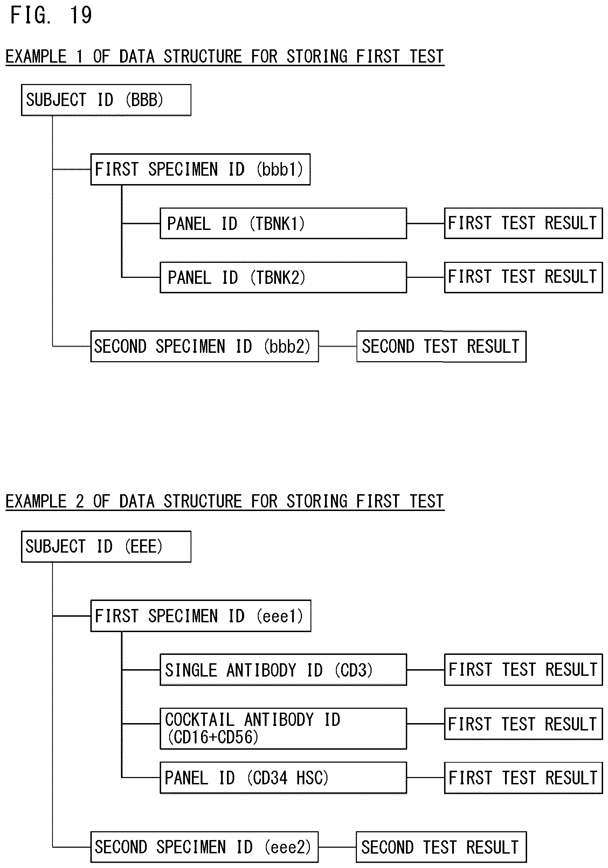

FIG. 19 schematically shows an example of a data structure for storing the first test result.

Example 1 shown in the upper part of FIG. 19 shows information to be stored into the database 102a in association with a subject ID when two panel IDs (TBNK1, TBNK2) are set in a test order for the first test. Example 1 corresponds to the first and second rows in the list display region 271 shown in FIG. 18.

Example 2 shown in the lower part of FIG. 19 shows information to be stored into the database 102a in association with a subject ID when one single antibody ID (CD3), one cocktail antibody ID (CD16+CD56), and one panel ID (CD34 HSC) are set in a test order for the first test. Example 2 corresponds to the fifth to seventh rows in the list display region 271 shown in FIG. 18.

The first test result regarding a single antibody includes a test result regarding an antibody of one type included in one antibody reagent. The first test result regarding a cocktail antibody includes respective test results regarding a plurality of types of antibodies included in one antibody reagent. The first test result of a panel includes respective test results regarding antibodies of various types included in a plurality of antibody reagents grouped in the panel.

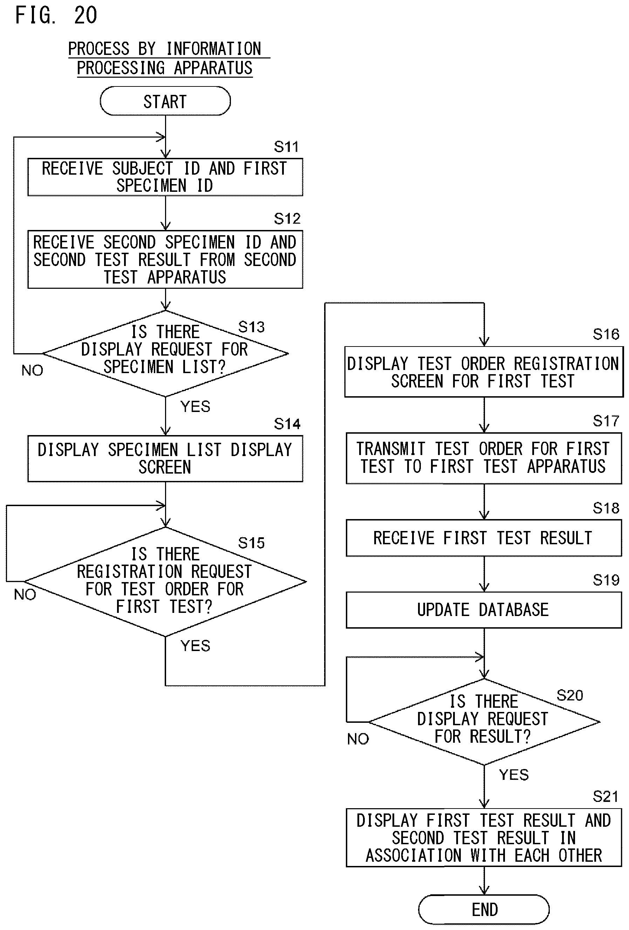

FIG. 20 is a flowchart showing a process performed by the information processing apparatus 10.

In step S11, the controller 101 of the information processing apparatus 10 causes the display unit 103 to display the inquiry screen 220 shown in FIG. 14 and receives a subject ID and a first specimen ID. In step S12, on the basis of the subject ID received through the inquiry screen 220, the controller 101 performs an inquiry to the second test apparatus 22, and receives a second specimen ID and a second test result corresponding to the subject ID, from the second test apparatus 22. At this time, the second test apparatus 22 transmits, to the information processing apparatus 10, the newest second specimen ID and second test result corresponding to the received subject ID, for example. The controller 101 stores, into the database 102a, the first specimen ID received in step S11 and the second specimen ID and the second test result received in step S12, in association with the subject ID received in step S11.

When the controller 101 has received a subject ID and a first specimen ID from, for example, another computer, the controller 101 may similarly perform an inquiry to the second test apparatus 22 and receive a second specimen ID and a second test result. In this case, operation by the operator through the inquiry screen 220 is not necessary.

In step S13, the controller 101 determines whether or not a display request for a specimen list has been received from the operator through the input unit 104. When the display request has not been received, the controller 101 returns the process to step S11. On the other hand, when the display request has been received, the controller 101 causes, in step S14, the display unit 103 to display the specimen list display screen 230 shown in FIG. 15.

In step S15, the controller 101 determines whether or not a registration request for a test order for the first test has been received from the operator through the registration button 232 in FIG. 15. When the registration request has been received, the controller 101 causes, in step S16, the display unit 103 to display the test order registration screen 240 for the first test shown in FIGS. 16, 17. The controller 101 receives the test order for the first test through the test order registration screen 240, and stores the test order for the first test into the storage 102.

In step S17, the controller 101 transmits the test order for the first test to the first test apparatus 21. The first test apparatus 21 performs the first test on the basis of the received test order for the first test, and generates first test results. In step S18, the controller 101 receives each first test result from the first test apparatus 21 in association with the first specimen ID. At this time, the controller 101 receives the first test result in association with the test identification information (the single antibody ID, the cocktail antibody ID, and the panel ID). In step S19, the controller 101 stores the first test result received in step S18, into the database 102a in association with the subject ID, the first specimen ID, the second specimen ID, and the second test result.

In steps S20, S21, the controller 101 performs the processes similar to those in steps S2, S3 in FIG. 5. That is, in step S20, the controller 101 determines whether or not a display request for a result has been inputted from the operator through the input unit 104. When the display request for the result has been inputted, the controller 101 causes, in step S21, the display unit 103 to display the first test result received in step S18 and the second test result received in step S12, in association with each other.

### <Effects of information processing method, information processing apparatus, and test system according to Embodiment 2>

In the specimen list display screen 230 shown in FIG. 15, a list of information in which the subject ID (information regarding the subject) is associated with the first specimen ID, the state of the first test, the second specimen ID, and the state of the second test (information regarding the first and second tests) is displayed. The controller 101 of the information processing apparatus 10 receives a test order for the first test with respect to the subject selected from the list display region 231 (list), through the test order registration screen 240 for the first test shown in FIGS. 16, 17. With this configuration, when the test order for the first test is inputted through the specimen list display screen 230, a database in which information regarding the first test and information regarding the second test are associated with each other can be constructed while using the information regarding the subject as a key.

In step S18 in FIG. 20, the controller 101 of the information processing apparatus 10 acquires the first test result with respect to the test order for the first test. In step S19, the controller 101 associates the subject ID (information regarding the subject) and the first and second test results with each other. According to this process, the first and second test results are also stored in association with the subject ID. Thus, result display in which the first and second test results are contrastable with each other can be easily executed.

In step S11 in FIG. 20, the controller 101 of the information processing apparatus 10 receives an input of a first specimen ID (first specimen identification information) that identifies a first specimen. In step S12, the controller 101 receives a second specimen ID (second specimen identification information) that corresponds to the first specimen ID (first specimen identification information) and that identifies a second specimen. In step S18, the controller 101 acquires first test results in association with the first specimen ID (first specimen identification information). According to this process, even when the first specimen and the second specimen respectively have different specimen IDs provided thereto, each first test result of the first specimen and the second test result of the second specimen can be easily associated with each other.

As shown in FIGS. 11, 12, an antibody reagent includes one or more antibodies and one or more fluorescent dyes (a combination corresponding to one row in the table). A plurality of types of antibody reagents are categorized for each container for preparing a measurement sample for the first test. In the first region 11 shown in FIGS. 6 to 9, a first test result is displayed for each category of a container for preparing a measurement sample for the first test. Specifically, in Embodiment 1 shown in FIG. 6, the first test results A to C are test results for respective categories of containers for preparing measurement samples for the first test. When the operator operates a tab, a button, a scroll bar, or the like in the first region 11, or when the operator operates a link button in the second region 12, the controller 101 of the information processing apparatus 10 causes a first test result to be switched and displayed, in the first region 11, for each category of a container for preparing a measurement sample for the first test. With this configuration, the first test result for each category is collectively displayed, and thus, when compared with a case where the first test results regarding all the antibody reagents are displayed in a list, the operator can smoothly refer to a target first test result.

In step S18 in FIG. 20, the controller 101 of the information processing apparatus 10 acquires first test results in association with a single antibody ID, a cocktail antibody ID, and a panel ID which are the test identification information that identifies the first test. With this configuration, a first test result can be easily acquired for each single antibody ID, cocktail antibody ID, and panel ID (test identification information). Accordingly, as shown in FIG. 10, the first test result can be smoothly stored into the database 102a for each single antibody ID, cocktail antibody ID, and panel ID.

As shown in FIGS. 11, 12, the single antibody ID, the cocktail antibody ID, and the panel ID which are the test identification information are each information indicating an antibody reagent to be used in the first test. In "test name" in the first region 11 shown in FIG. 6, a single antibody ID, a cocktail antibody ID, or a panel ID is displayed as the test identification information. As shown in FIG. 6, when information indicating the antibody reagent used in the first test is displayed together with the first test result, the operator can smoothly grasp the antibody reagent used in the first test, and thus, can smoothly and appropriately perform evaluation based on the first test result and the second test result. When a panel ID is displayed in the first region 11, information on a plurality of antibody reagents grouped by the panel ID may also be displayed.

In the test order registration screen 240 shown in FIGS. 16, 17, a single antibody ID, a cocktail antibody ID, and a panel ID are displayed as the test identification information. Accordingly, the operator can smoothly grasp the antibody reagent to be used in the first test, and thus, can appropriately register the test order.

As shown in FIG. 13, the operator can set as desired the types of a plurality of antibody reagents associated with a panel ID (test identification information), through the panel editing screen 210. With this configuration, a necessary first test can be freely set in accordance with the facility or the like that uses the first test apparatus 21. As shown in FIGS. 6 to 9, first test results corresponding to the panel set by the operator are displayed together with the second test result in the test result screen 110, and thus, the operator can perform a further detailed analysis.

In the first test, analysis regarding an antigen present at least at one of a cell surface and inside of a cell is performed. On the other hand, in the second test, analysis regarding a component different from antigens is performed. With this configuration, the operator can evaluate the first test result with reference to the result of analysis (e.g., analysis regarding cell morphology, intracellular granules, intracellular nucleic acid, etc.) regarding a component, different from antigens, that is difficult to be analyzed in the first test. For example, the first test result can be evaluated on the basis of whether or not the first test result is a result indicating the same directionality as the second test result.

### <Modification 1 of Embodiment 2>

In Embodiment 2, when registering a test order for the first test, the second test result may be displayed on the display unit 103.

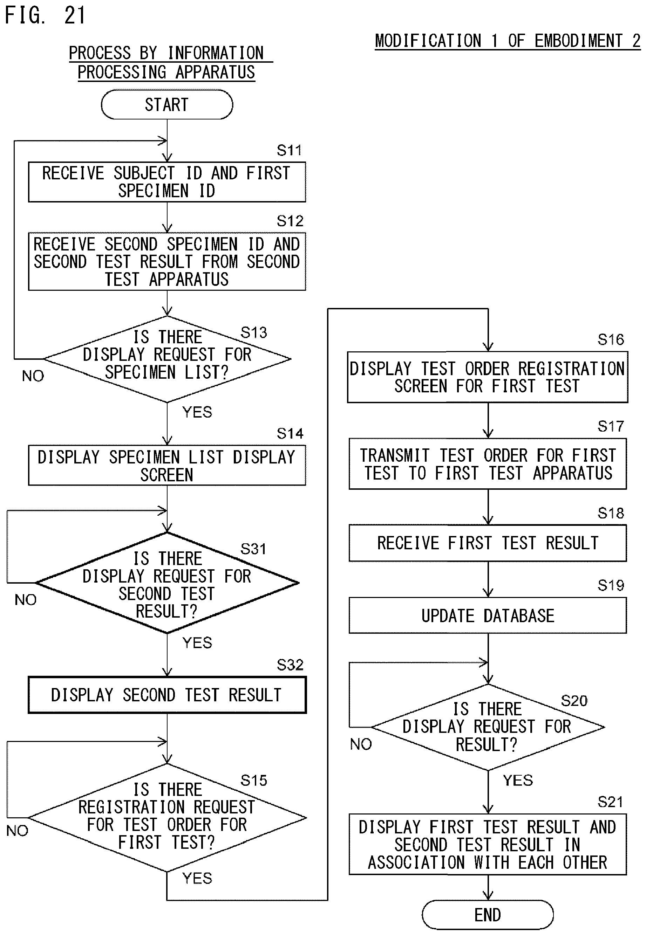

FIG. 21 is a flowchart showing a process performed by the information processing apparatus 10, according to the present modification. In the process in FIG. 21, steps S31, S32 are added between step S14 and step S15 when compared with FIG. 20. Hereinafter, processes different from those in FIG. 20 will be described.

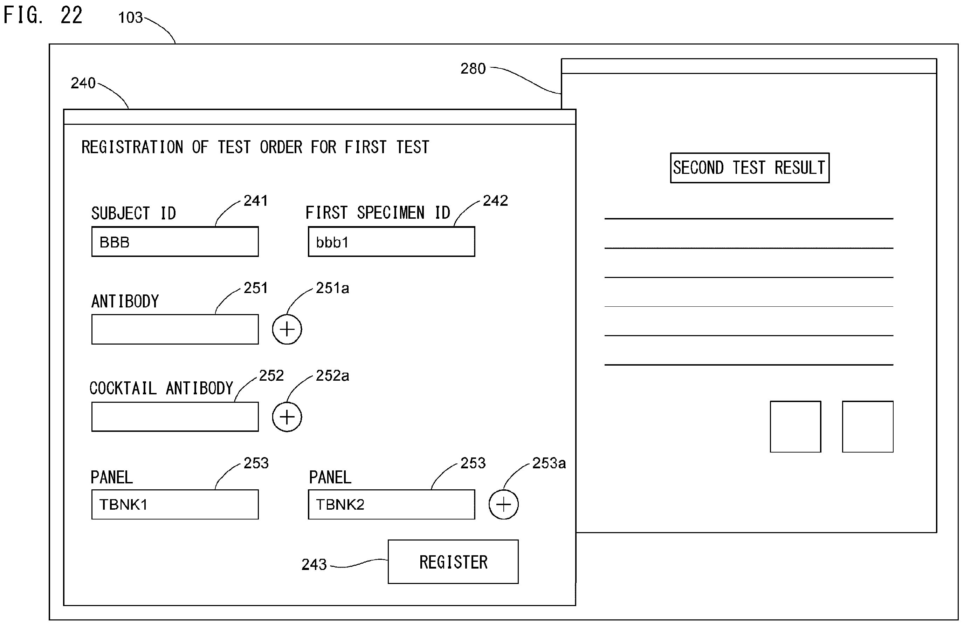

In step S31, the controller 101 of the information processing apparatus 10 determines whether or not a display request for the second test result has been received from the operator through the input unit 104. The operator inputs a display request for the second test result by operating a character string of "tested" in the item of the state of the target second test in FIG. 15, for example. When having received the display request for the second test result, the controller 101 causes, in step S32, the display unit 103 to display a display screen 280 for the second test result shown in FIG. 22, on the basis of the second test result received in step S12.

Then, when a registration request for a test order for the first test is received from the operator, the controller 101 causes, in step S16, the display unit 103 to display the test order registration screen 240 for the first test shown in FIGS. 16, 17, while maintaining the display of the display screen 280 displayed in step S32.

FIG. 22 schematically shows a state where the display screen 280 for the second test result is displayed together with the test order registration screen 240 for the first test, on the display unit 103 of the information processing apparatus 10.

The second test result is displayed in the display screen 280 for the second test result. When steps S32, S16 in FIG. 21 have been executed, two screens are displayed so as to be arranged in the display unit 103 as shown in FIG. 22. Accordingly, while looking at the second test result, the operator can determine what test is necessary in the first test and can smoothly register an order for the first test through the test order registration screen 240 for the first test.

When the test order registration screen 240 for the first test is displayed, the display screen 280 for the second test result need not necessarily be simultaneously displayed. For example, a button for displaying the test order registration screen 240 for the first test may be provided in the display screen 280 for the second test result, and when this button is operated, the test order registration screen 240 for the first test may be displayed instead of the display screen 280 for the second test result.

### <Effects of information processing method, information processing apparatus, and test system according to Modification 1 of Embodiment 2>

As shown in FIG. 22, the operator selects an antibody reagent in accordance with the result of the second test. In step S18 in FIG. 21, the controller 101 of the information processing apparatus 10 acquires a first test result obtained through the first test using the antibody reagent selected in accordance with the second test result. According to this process, the first test result can be acquired by using the antibody reagent according to the second test result, and highly reliable information that can be utilized in disease diagnosis can be obtained.

In step S31 in FIG. 21, the controller 101 displays the second test result before acquiring the first test result. In step S 16, the controller 101 receives a test order for the first test after displaying the second test result. According to this process, it is possible to display the second test result before performing the first test, refer to the second test result, and then create a test order for the first test.

Through steps S32, S16 in FIG. 21, the display screen 280 for the second test result and the test order registration screen 240 for the first test (reception screen for receiving the test order for the first test) are displayed in association with each other. Specifically, the display screen 280 for the second test result and the test order registration screen 240 for the first test are simultaneously displayed, or alternatively, after display of the display screen 280 for the second test result, the test order registration screen 240 for the first test is displayed instead of the display screen 280 for the second test result. According to this process, with reference to the second test result, the operator can easily set a test order for the first test including an antibody reagent of an appropriate type for a test with respect to a disease that can be assumed from the second test result.

### <Modification 2 of Embodiment 2>

In Embodiment 2, the test order for the first test is set by the operator through the test order registration screen 240 for the first test, but the test order for the first test may be automatically set by a computer.

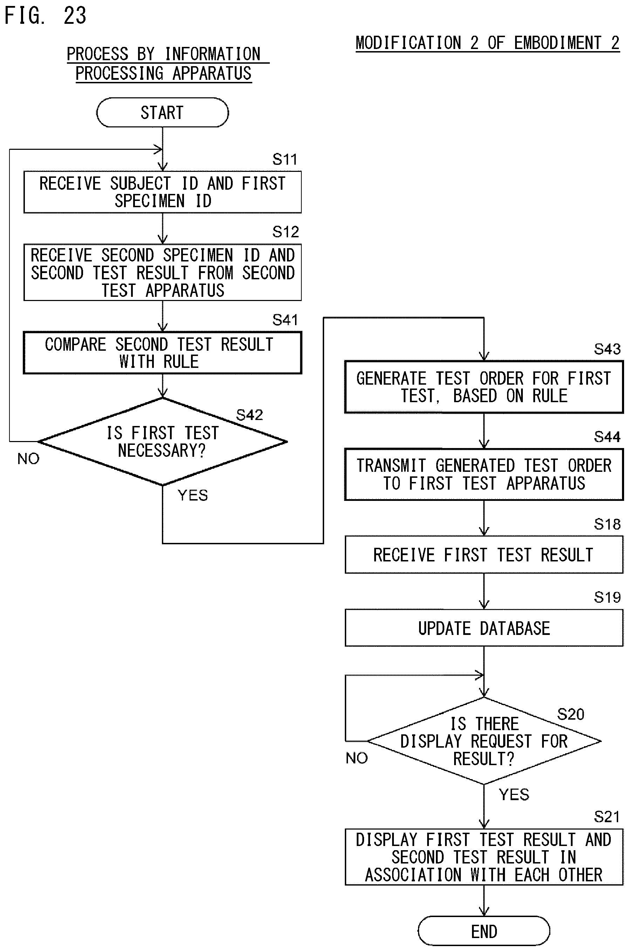

FIG. 23 is a flowchart showing a process performed by the information processing apparatus 10, according to the present modification. In the process in FIG. 23, steps S41 to S44 are added in place of steps S13 to S 17 when compared with FIG. 20. Hereinafter, processes different from those in FIG. 20 will be described.

In step S41, the controller 101 of the information processing apparatus 10 compares the second test result received in step S12 with rules for setting the first test. The rules for setting the first test are stored in advance in the storage 102.

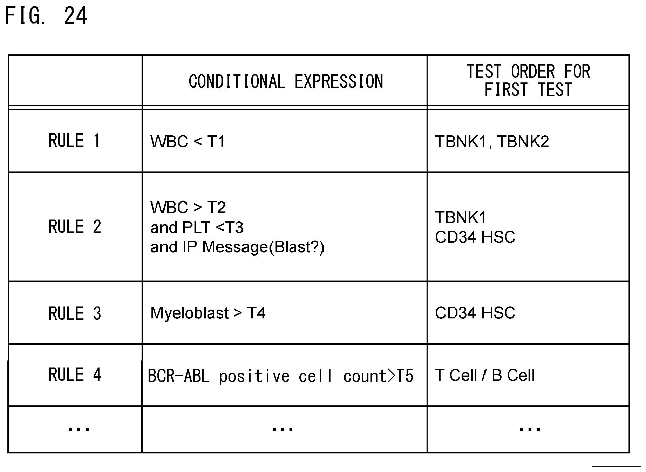

FIG. 24 is a table showing an example of the rules for setting the first test.

As shown in Rule 1, when WBC (white blood cell count) included in the second test result is smaller than a predetermined threshold T1, the controller 101 sets panel IDs "TBNK1" and "TBNK2" as a test order for the first test. As shown in Rule 2, when WBC (white blood cell count) included in the second test result is larger than a predetermined threshold T2, PLT (platelet count) included in the second test result is smaller than a predetermined threshold T3, and a message of "Blast?" (blast cell present?) is included in the second test result, the controller 101 sets panel IDs "TBNK1" and "CD34 HSC" as a test order for the first test. As shown in Rule 3, when Myeloblast (myeloblast count) included in the second test result is larger than a predetermined threshold T4, the controller 101 sets a panel ID "CD34 HSC" as a test order for the first test. As shown in Rule 4, when BCR-ABL positive cell count included in the second test result is larger than a predetermined threshold T5, the controller 101 sets a panel ID "T Cell/B Cell" as a test order for the first test.

With reference back to FIG. 23, in step S42, the controller 101 determines whether or not it is necessary to set a test order for the first test shown in the rules, on the basis of the comparison result in step S41.

When the first test is not necessary, the controller 101 returns the process to step S11. On the other hand, when the first test is necessary, the controller 101 generates, in step S43, a test order for the first test, on the basis of the rules as shown in FIG. 24. When the second test result matches a plurality of the rules, a test order for the first test corresponding to the plurality of the rules is generated. In step S44, the controller 101 transmits the test order for the first test generated in step S43, to the first test apparatus 21.

### <Effects of information processing method, information processing apparatus, and test system according to Modification 2 of Embodiment 2>

In steps S41 to S43 in FIG. 23, the controller 101 of the information processing apparatus 10 generates a test order for the first test with respect to the subject, on the basis of the second test result. According to this process, since a test order for the first test is automatically generated, the work by the operator of setting a test order for the first test with reference to the second test result can be omitted.

### <Modification 3 of Embodiment 2>

In Embodiment 2, the information processing apparatus 10 performs an inquiry to the second test apparatus 22 on the basis of the subject ID, and the second test apparatus 22 transmits the newest second specimen ID and second test result corresponding to the subject ID, to the information processing apparatus 10. In contrast to this, in the present modification, the information processing apparatus 10 receives the second specimen ID that should be received from the second test apparatus 22, from another computer, and on the basis of the received second specimen ID, receives the second test result from the second test apparatus 22.

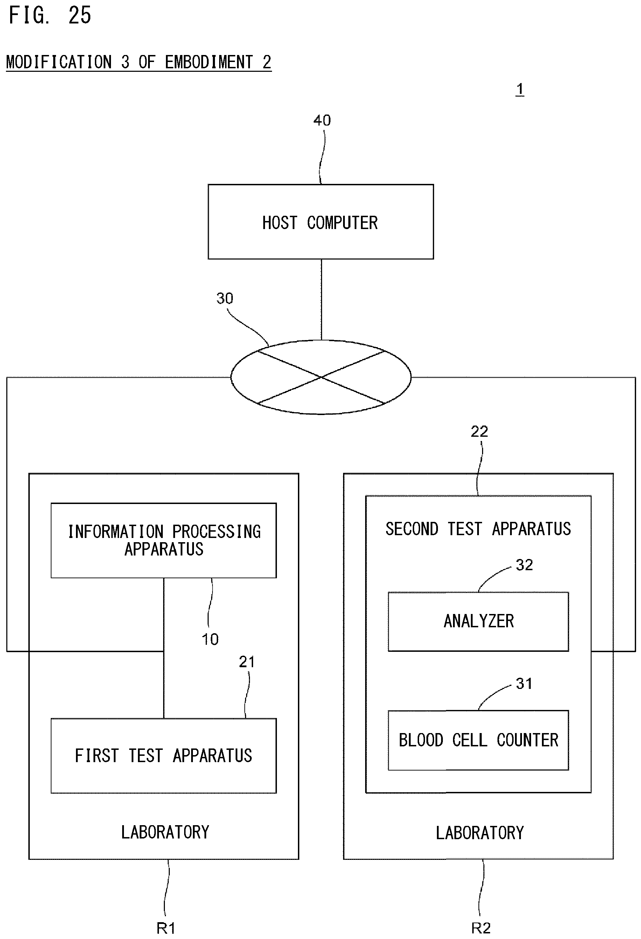

FIG. 25 is a block diagram showing a configuration of the test system 1 according to the present modification.

When compared with FIG. 2, the test system 1 of the present modification further includes a host computer 40, and the second test apparatus 22 includes a blood cell counter 31 and an analyzer 32. The host computer 40 is communicably connected to the information processing apparatus 10, the first test apparatus 21, and the second test apparatus 22, through the network 30. The information processing apparatus 10 of the present modification is disposed in the laboratory R1.

The host computer 40 has stored, in a storage in advance, subject IDs, first specimen IDs, and second specimen IDs in association with each other. When the host computer 40 has received an inquiry based on a subject ID and a first specimen ID from the information processing apparatus 10, the host computer 40 transmits a corresponding second specimen ID to the information processing apparatus 10. The blood cell counter 31 acquires measurement data regarding blood cells by preparing a measurement sample on the basis of a second specimen, and measuring the measurement sample. The analyzer 32 transmits an instruction to perform the second test, to the blood cell counter 31, performs analysis on the basis of the measurement data obtained by the blood cell counter 31, and generates a second test result corresponding to the subject ID and the second specimen ID.

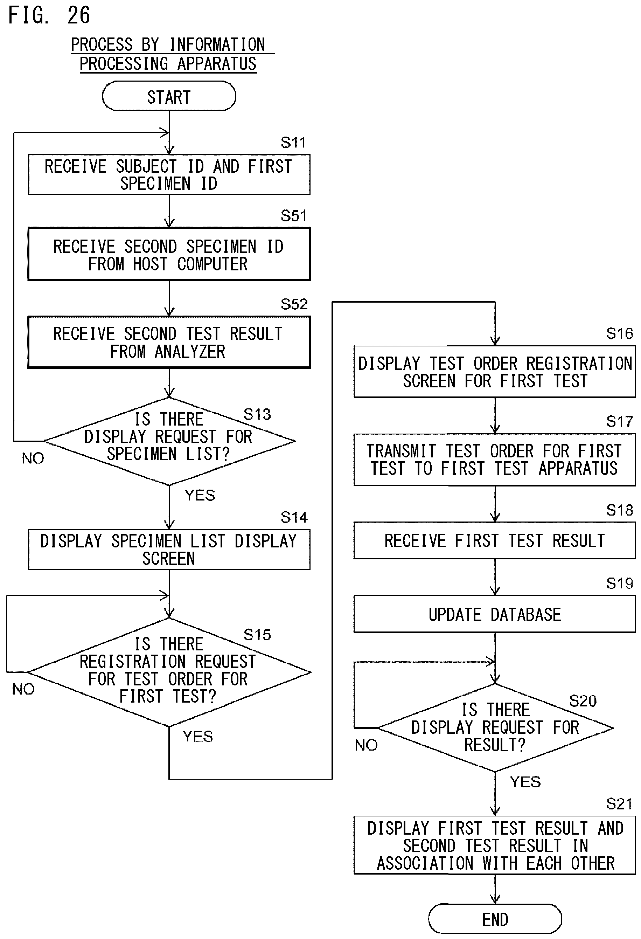

FIG. 26 is a flowchart showing a process performed by the information processing apparatus 10, according to the present modification. In the process in FIG. 26, steps S51, S52 are added in place of step S12 when compared with FIG. 20. Hereinafter, processes different from those in FIG. 20 will be described.

In step S51, the controller 101 of the information processing apparatus 10 performs an inquiry to the host computer 40 on the basis of the subject ID and the first specimen ID received in step S11, and receives a second specimen ID corresponding to the subject ID and the first specimen ID from the host computer 40. In step S52, the controller 101 performs an inquiry to the analyzer 32 of the second test apparatus 22 on the basis of the subject ID and the second specimen ID received in step S51, and receives a second test result corresponding to the subject ID and the second specimen ID from the analyzer 32.

The controller 101 stores, into the database 102a shown in FIG. 10, the first specimen ID received in step S11, the second specimen ID received in step S51, and the second test result received in step S52, in association with the subject ID received in step S11.

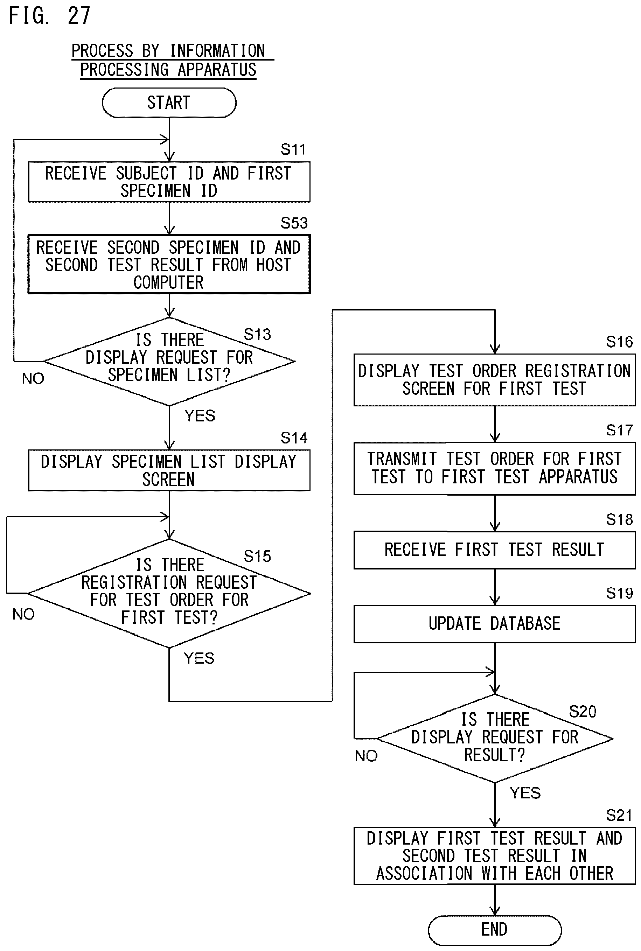

The host computer 40 may receive the second specimen ID and the second test result from the analyzer 32 when the second test has been performed by the second test apparatus 22, and may store the received second test result into a storage in association with the second specimen ID. When the host computer 40 stores the second test result, step S53 is added in place of steps S51, S52 in FIG. 26, as shown in FIG. 27. In step S53, the controller 101 performs an inquiry to the host computer 40 on the basis of the subject ID and the first specimen ID, and receives a corresponding second specimen ID and a corresponding second test result from the host computer 40.

Alternatively, the host computer 40 may perform the above-described process performed by the information processing apparatus 10. For example, when the host computer 40 stores the first test result and the second test result in association with the subject ID, the first specimen ID, and the second specimen ID, the host computer 40 may generate screen data in which the first test result and the second test result are associated with each other. In this case, when receiving a display request in step S20 in FIG. 27, the controller 101 of the information processing apparatus 10 transmits a request instruction for image data to the host computer 40. Upon receiving the request instruction for image data, the host computer 40 generates image data in which the first test result and the second test result are associated with each other, and transmits the generated image data to the information processing apparatus 10. In step S21 in FIG. 27, the controller 101 of the information processing apparatus 10 causes the display unit 103 to display the received image data.

### <Effects of information processing method, information processing apparatus, and test system according to Modification 3 of Embodiment 2>

Since the host computer 40 has stored, in a storage in advance, subject IDs, first specimen IDs, and second specimen IDs in association with each other, the controller 101 of the information processing apparatus 10 can assuredly acquire a second specimen ID in one-to-one correspondence, by performing an inquiry to the host computer 40 on the basis of a subject ID and a first specimen ID. Accordingly, the second test result to be contrasted can be displayed together with the first test result in the test result screen 110.

As described with reference to FIG. 27, when the host computer 40 collectively stores the second test result associated with the second specimen ID, there is no need for the first test apparatus 21 to perform an inquiry to the second test apparatus 22 in order to acquire the second test result. In this case, the first test apparatus 21 can efficiently acquire the second test result from the host computer 40, and there is no need for the second test apparatus 22 to reply to the inquiry from the first test apparatus 21.

### <Embodiment 3>

In Embodiment 3, the second test apparatus 22 is a blood cell counter. In Embodiment 3, detailed configurations of the first test apparatus 21 and the second test apparatus 22 will be described. Unless otherwise specified, the configuration and the process in Embodiment 3 are similar to those in Embodiment 1.

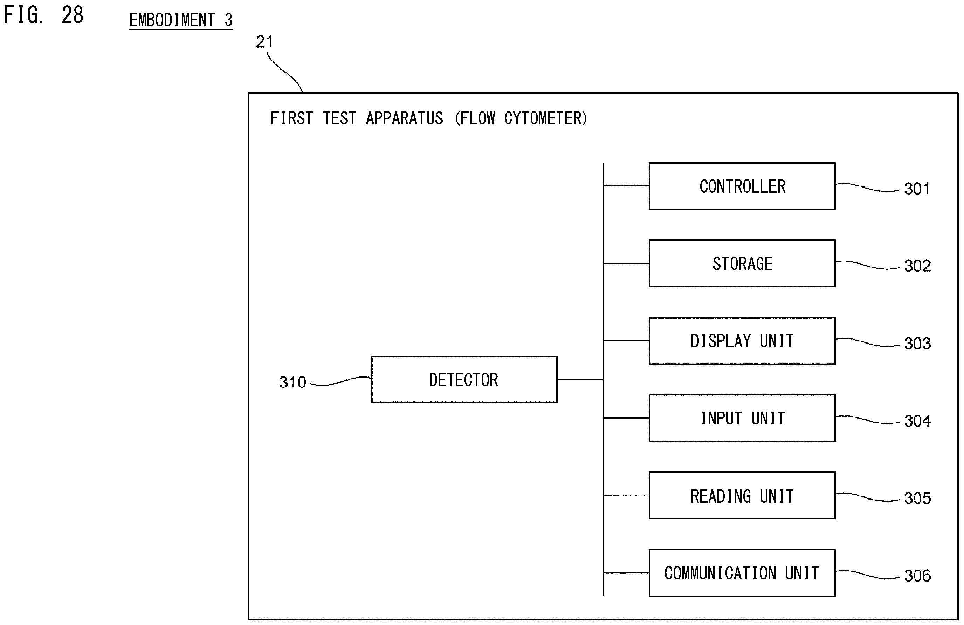

FIG. 28 is a block diagram showing a configuration of the first test apparatus 21 according to Embodiment 3.

The first test apparatus 21 includes a controller 301, a storage 302, a display unit 303, an input unit 304, a reading unit 305, a communication unit 306, and a detector 310.

The controller 301 is implemented by an FPGA or a CPU, for example. The storage 302 is implemented by a ROM, a RAM, an SSD, an HDD, or the like, for example. The controller 301 receives a signal outputted by each component of the first test apparatus 21, and controls each component of the first test apparatus 21.

The display unit 303 is implemented by a liquid crystal display, for example. The input unit 304 is implemented by a keyboard and a mouse, for example. The reading unit 305 is implemented by a barcode reader, for example. The reading unit 305 reads a barcode from a label attached to a container containing a first specimen, and acquires a subject ID and a first specimen ID. The communication unit 306 is a communication interface based on the Ethernet standard, for example. The controller 301 communicates with the information processing apparatus 10 and the second test apparatus 22 through the communication unit 306.

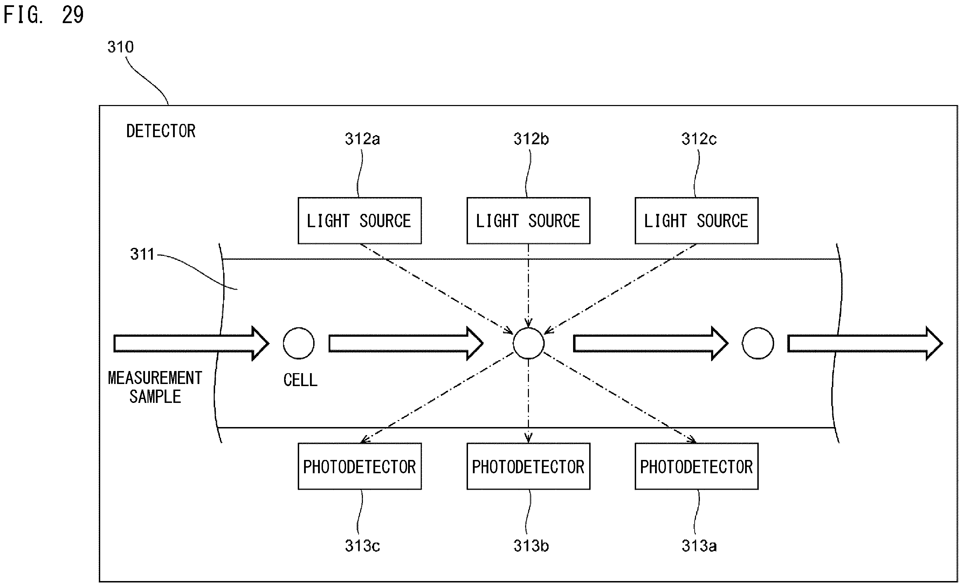

FIG. 29 schematically shows a configuration of the detector 310.

The detector 310 includes a flow cell 311, three light sources 312a, 312b, 312c, and three photodetectors 313a, 313b, 313c.

A measurement sample is caused to flow in the flow cell 311. The measurement sample is prepared in accordance with a test order for the first test, from a first specimen supplied to the first test apparatus 21. Preparation of the measurement sample is performed by hand, a sample preparation unit or a specimen pretreatment device provided to the first test apparatus 21, or the like, for example. Through preparation of the measurement sample, an antigen present at least at one of a cell surface and inside of a cell serving as a target in the measurement sample is fluorescence-labeled by an antibody reagent as shown in FIGS. 11, 12.

The three light sources 312a, 312b, 312c apply lights having different wavelengths from each other to each cell in the measurement sample flowing in the flow cell 311. The three light sources 312a, 312b, 312c are configured such that emitted lights cause a fluorescent dye to excite fluorescence. Accordingly, fluorescence is generated from the fluorescent dye labeling the cell. The three photodetectors 313a, 313b, 313c respectively receive fluorescence generated from the lights from the three light sources 312a, 312b, 312c, and output detection signals according to the received light intensities. The light sources 312a, 312b, 312c are implemented by semiconductor laser light sources, for example, and the photodetectors 313a, 313b, 313c are implemented by avalanche photodiodes, for example.

In FIG. 29, the three pairs of a light source and a photodetector are provided. However, one, two, four, or more pairs of a light source and a photodetector may be provided.

With reference back to FIG. 28, the controller 301 performs analysis of the first specimen on the basis of the detection signals acquired in the detector 310, on the basis of a program stored in the storage 302, and generates a first test result. The controller 301 stores the generated first test result into the storage 302, and transmits, to the information processing apparatus 10, the first test result in association with a subject ID and a first specimen ID.

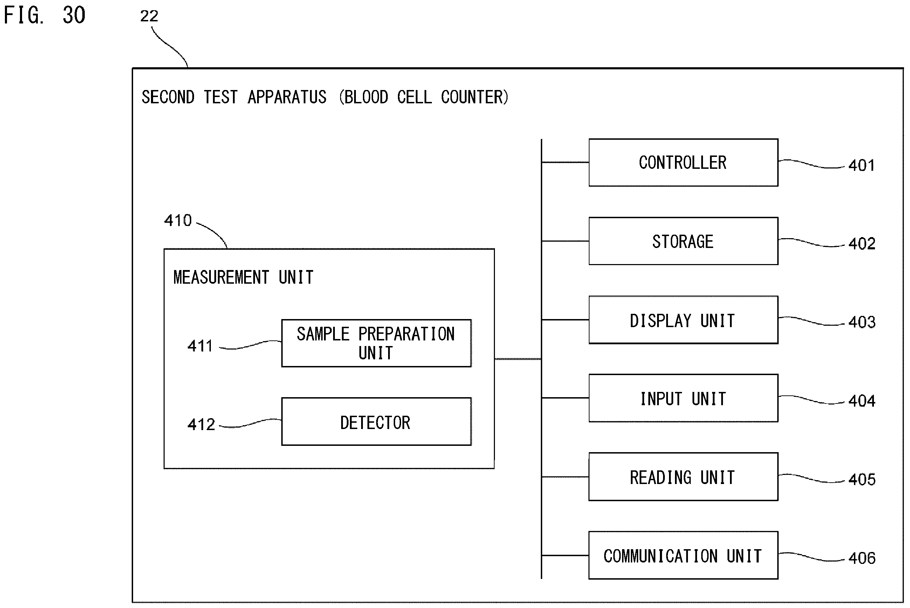

FIG. 30 is a block diagram showing a configuration of the second test apparatus 22.

The second test apparatus 22 of the present embodiment is a blood cell counter as described above. The second test apparatus 22 includes a controller 401, a storage 402, a display unit 403, an input unit 404, a reading unit 405, a communication unit 406, and a measurement unit 410.

The controller 401 is implemented by an FPGA or a CPU, for example. The storage 402 is implemented by a ROM, a RAM, an SSD, an HDD, or the like, for example. The controller 401 receives a signal outputted by each component of the second test apparatus 22, and controls each component of the second test apparatus 22.

The display unit 403 is implemented by a liquid crystal display, for example. The input unit 404 is implemented by a keyboard and a mouse, for example. The reading unit 405 is implemented by a barcode reader, for example. The reading unit 405 reads a barcode from a label attached to a container containing a second specimen, and acquires a subject ID and a second specimen ID. The communication unit 406 is a communication interface based on the Ethernet standard, for example. The controller 401 communicates with the information processing apparatus 10 and the first test apparatus 21 through the communication unit 406.

The measurement unit 410 includes a sample preparation unit 411 and a detector 412. The sample preparation unit 411 mixes a second specimen and a reagent on the basis of a test order for the second test, and prepares a plurality of types of measurement samples. The detector 412 measures each measurement sample prepared in the sample preparation unit 411, and acquires a detection signal. The detector 412 is implemented by an optical detector, an electric-resistance-type detector, a hemoglobin detector, or the like, for example. The optical detector measures blood cells by a flow cytometry method. The electric-resistance-type detector measures blood cells by a sheath flow DC detection method. The hemoglobin detector measures hemoglobin by an SLS-hemoglobin method.

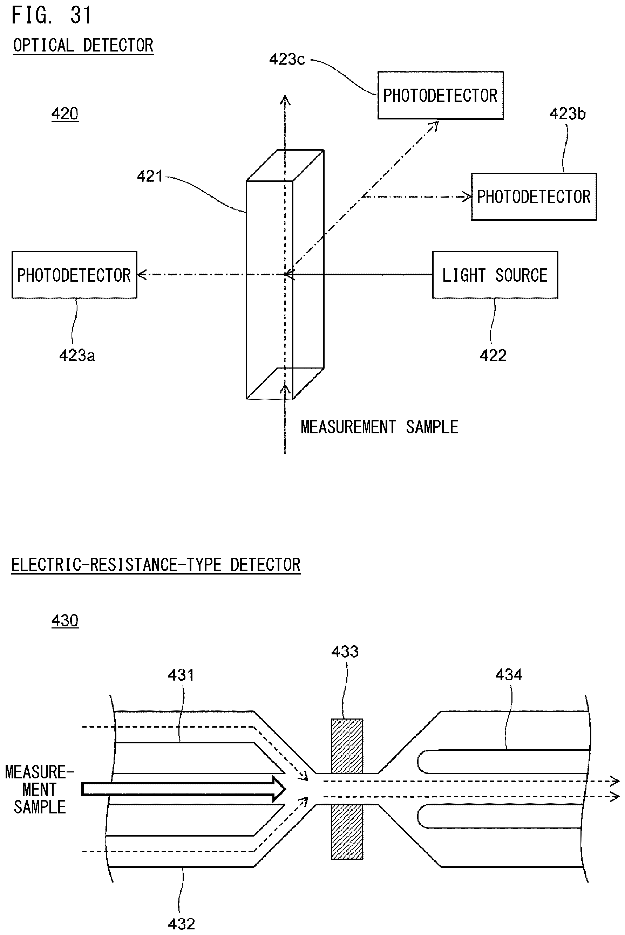

FIG. 31 schematically shows configurations of an optical detector 420 and an electric-resistance-type detector 430.

As shown in the upper part of FIG. 31, the optical detector 420 includes a flow cell 421, a light source 422, and photodetectors 423a, 423b, 423c.

A measurement sample prepared in the sample preparation unit 411 is caused to flow in the flow cell 421. The light source 422 applies light having a predetermined wavelength to each blood cell in the measurement sample flowing in the flow cell 421. Accordingly, from a particle, forward scattered light is generated forwardly, and side scattered light and fluorescence are generated sideways. The three photodetectors 423a, 423b, 423c respectively receive forward scattered light, side scattered light, and fluorescence, and output detection signals according to the received light intensities. The light source 422 is implemented by a semiconductor laser light source, for example, and the photodetectors 423a, 423b, 423c are implemented by avalanche photodiodes, for example.

As shown in the lower part of FIG. 31, the electric-resistance-type detector 430 includes a sample nozzle 431, a chamber 432, an aperture 433, and a collection tube 434.

The sample nozzle 431 sends out a measurement sample, together with a sheath liquid, in a direction toward the aperture 433. The chamber 432 has a tapered shape that becomes thinner toward the aperture 433. The measurement sample advances through the aperture 433 to the collection tube 434. Blood cells contained in the measurement sample pass through the aperture 433 in a state of being aligned in one line. Direct current is supplied between electrodes of the aperture 433 and a detection signal according to change in direct current resistance when the measurement sample passes through the aperture 433 is outputted. Since the direct current resistance increases when a blood cell in the measurement sample passes through the aperture 433, the detection signal reflects information on the blood cell passing through the aperture 433.

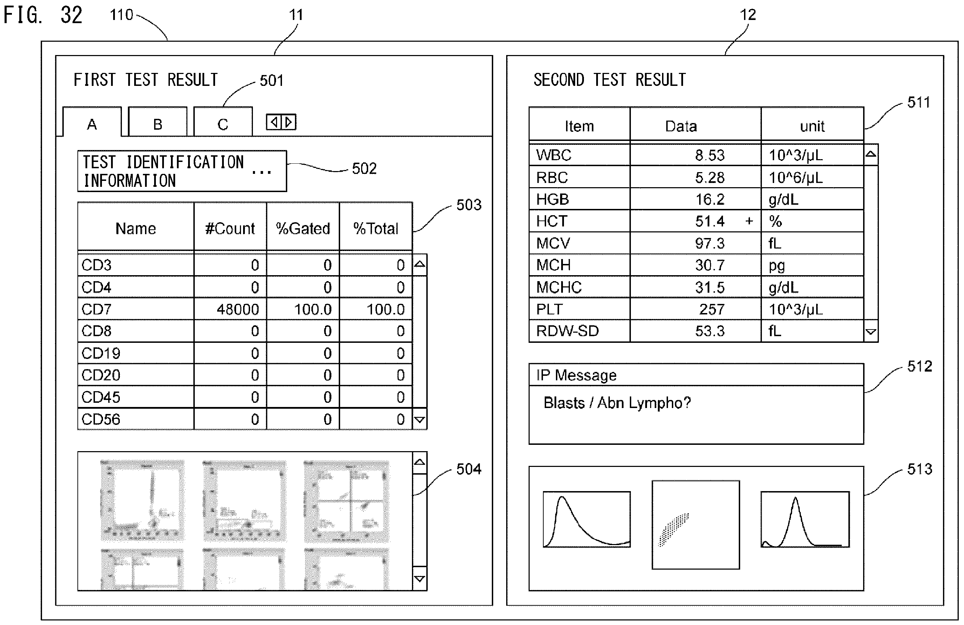

FIG. 32 schematically shows a configuration of the test result screen 110 displayed on the display unit 103 of the information processing apparatus 10.

Similar to Embodiment 1 shown in FIGS. 6 to 9, the test result screen 110 includes the first region 11 for displaying each of a plurality of first test results obtained on the basis of a first specimen; and the second region 12 for displaying a second test result obtained on the basis of a second specimen associated with the first specimen. The first test result in the first region 11 and the second test result in the second region 12 correspond to each other. When the first specimen and the second specimen correspond to each other (e.g., when the first specimen and the second specimen have been acquired at a short time interval), the first test result obtained from the first specimen and the second test result obtained from the second specimen also correspond to each other. That is, in the test result screen 110, two test results obtained from a first specimen and a second specimen corresponding to each other are displayed so as to be arranged side by side.

The first region 11 includes a plurality of tabs 501, a test name display region 502, a numerical value display region 503, and a graph display region 504.

Each tab 501 is provided so as to correspond to the category of the single antibody, the cocktail antibody, and the panel. By operating a tab 501, the operator can cause a desired first test result, out of a plurality of first test results, to be displayed in the first region 11. In the test name display region 502, a test name corresponding to the operated tab 501 is displayed. The test name is one of a single antibody ID, a cocktail antibody ID, and a panel ID. The test name may be a name corresponding to each ID.

In the numerical value display region 503, a first test result obtained through the first test corresponding to the test name display region 502 is displayed. In the numerical value display region 503, the count and the ratio, of blood cells expressing each antigen, obtained by demarcating blood cells plotted on the scattergrams in the graph display region 504, by a region corresponding to the type of the antigen (e.g., CD3, CD4, CD7, CD8, CD16, CD19, CD20, CD34, CD45, CD56, etc.) are displayed. In the graph display region 504, scattergrams obtained through the first test corresponding to the test name display region 502 are displayed.

The second region 12 includes a numerical value display region 511, a message display region 512, and a graph display region 513. The second test result shown in the second region 12 corresponds to the first test result displayed in the first region 11.

In the numerical value display region 511, values (e.g., red blood cell count, hematocrit value, mean corpuscular volume, etc.) showing the states of blood cells obtained on the basis of the histograms in the graph display region 513, the count and the ratio for each type of blood cell obtained by demarcating blood cells plotted on the scattergrams in the graph display region 513 by a region corresponding to the type (e.g., red blood cell, white blood cell, platelet, etc.), and the like are displayed. In the message display region 512, a message generated by the second test apparatus 22 at the time of analysis based on the second test is displayed. In the message display region 512, an item (e.g., the type of blood cell), of the second test result, that shows an abnormal value is displayed, for example. In the message display region 512 shown as an example in FIG. 32, "Blasts/Abn Lympho?" is displayed as a message suggesting that many blast cells or abnormal lymphocytes are present. In the graph display region 513, histograms and scattergrams obtained through the second test are displayed.

### <Effects of information processing method, information processing apparatus, and test system according to Embodiment 3>

With the test result screen 110 shown in FIG. 32, with reference to the first test result including the analysis result of surface antigens by the first test apparatus 21 and the second test result including the analysis result of blood cells by the second test apparatus 22, the operator can evaluate the validity of the first test result and a possibility of a specific disease. Accordingly, the efficiency of the test by the first test apparatus 21 (flow cytometer) can be increased.

For example, when the white blood cell count according to the second test result is small, there is a possibility that the subject has HIV. In this case, for example, a test order for the first test for obtaining the CD4-positive T-lymphocyte count is created, and the first test is performed. When the CD4-positive T-lymphocyte count according to the first test result is small similar to the white blood cell count according to the second test result, the operator can determine that the validity of the first test result is high, and can validate the first test result. Since the white blood cell count according to the second test result is small and the CD4-positive T-lymphocyte count according to the first test result is small, the operator can determine that the possibility that the subject has HIV is high.

When, in the second region 12, a fact that the white blood cell count is large is displayed in the numerical value display region 511 and a message suggesting that many blast cells are present is displayed in the message display region 512, the operator can grasp a possibility that the subject has AML (acute myeloid leukemia). When the operator has determined that a first test result necessary for determining contract of AML is not displayed in the first region 11 displayed in contrast, the operator can create a test order for the first test including a necessary antibody reagent, through the test order registration screen 240 in FIGS. 16, 17, and can cause the first test apparatus 21 to perform the first test again. With reference to the second test result and the first test result acquired again which are displayed in contrast, the operator can determine whether or not the first test result is valid (e.g., whether or not the first test result reinforces the possibility of contract of AML) as a basis for the possibility of contract of AML obtained in the second test result.

As described above, with reference to the numerical value display region 511 and the message display region 512 included in the second region 12, the operator can grasp a possibility of a predetermined disease. With reference to such a second region 12 and the first region in contrast, the operator can evaluate the validity of the first test result efficiently and in a simple manner in terms of an additional test order as described above.

### <Modification 1 of Embodiment 3>

In Embodiment 3, the second test apparatus 22 is a blood cell counter, but not limited thereto, the second test apparatus 22 may be a blood cell image analyzer that captures a blood cell image and performs analysis of blood cells on the basis of the captured blood cell image. The second test apparatus 22 in this case is also an apparatus that performs a hematology test on the second specimen.

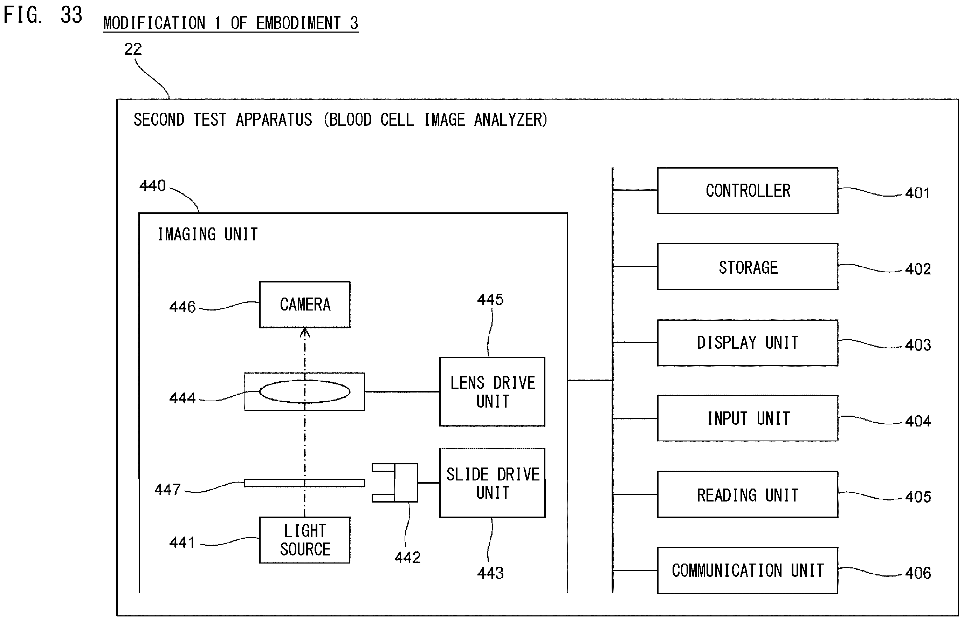

FIG. 33 is a block diagram showing a configuration of the second test apparatus 22 according to the present modification.

When compared with Embodiment 3 shown in FIG. 30, the second test apparatus 22 of the present modification includes an imaging unit 440 in place of the measurement unit 410. The imaging unit 440 includes a light source 441, a holder 442, a slide drive unit 443, an objective lens 444, a lens drive unit 445, and a camera 446.

The light source 441 emits light having a predetermined wavelength. The light emitted from the light source 441 is applied to a measurement sample placed on a smear slide 447. The measurement sample placed on the smear slide 447 has been prepared in advance on the basis of a second specimen collected from the subject, and includes blood cells. The holder 442 holds the smear slide 447 having placed thereon the measurement sample including cells of which an image is to be captured. The slide drive unit 443 moves the holder 442, thereby moving the smear slide 447 in three axial directions orthogonal to each other.

The objective lens 444 condenses light having passed through blood cells in the measurement sample. The lens drive unit 445 moves the objective lens 444 in the optical axis direction. The camera 446 receives, at a light receiving surface, the light condensed by the objective lens 444, and outputs image information corresponding to the distribution of light on the light receiving surface. The camera 446 is a CCD image sensor, for example. The controller 401 generates a blood cell image on the basis of the image information outputted from the camera 446 and classifies the cells on the basis of the blood cell image.

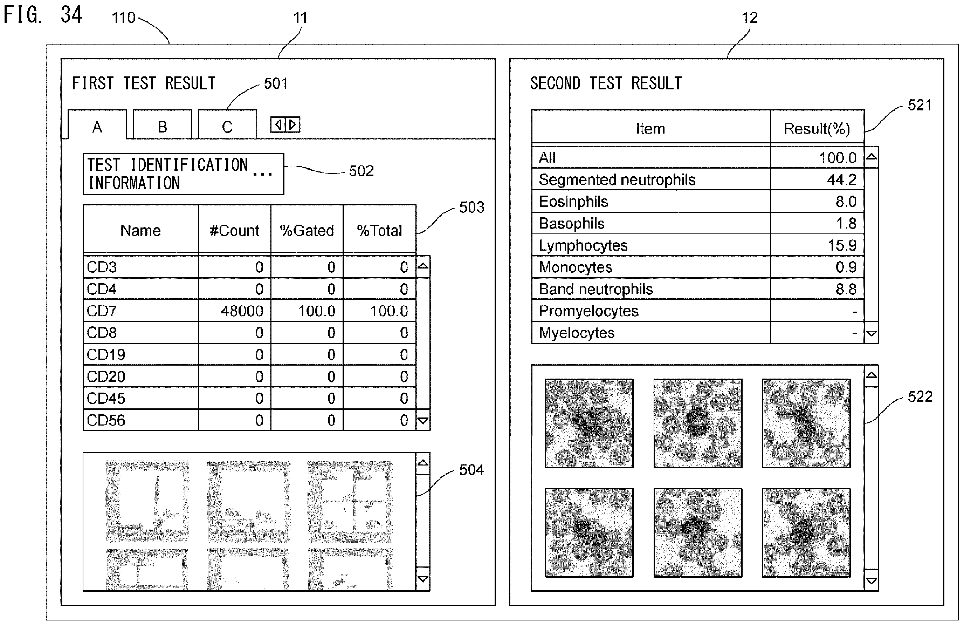

FIG. 34 schematically shows a configuration of the test result screen 110 displayed on the display unit 103 of the information processing apparatus 10, according to the present modification.

When compared with Embodiment 3 shown in FIG. 32, the test result screen 110 of the present modification is changed in the display in the second region 12. The second region 12 of the present modification includes a numerical value display region 521 and an image display region 522.

In the numerical value display region 521, the ratio for each type of blood cell (e.g., red blood cell, white blood cell, platelet, etc.) obtained through the analysis of the blood cell images in the image display region 522 is displayed. In the image display region 522, blood cell images captured in the second test are displayed. When a character string indicating the type of blood cell in the numerical value display region 521 is operated by the operator, a blood cell image corresponding to the type of blood cell having been operated is displayed in the image display region 522. For example, when "Lymphocytes" in the numerical value display region 521 is selected by the operator, a blood cell image corresponding to lymphocyte is displayed in the image display region 522.

### <Effects of information processing method, information processing apparatus, and test system according to Modification 1 of Embodiment 3>

With the test result screen 110 shown in FIG. 34, with reference to the first test result including the analysis result of surface antigens by the first test apparatus 21 and the second test result including the analysis result of blood cell images by the second test apparatus 22, the operator can evaluate the validity of the first test result and a possibility of a specific disease. Accordingly, the efficiency of the test by the first test apparatus 21 (flow cytometer) can be increased.

For example, when there are many myeloblasts according to the second test result, there is a possibility that the subject has hematologic malignancy. In this case, for example, a test order for the first test for obtaining the CD34-positive cell count is created, and the first test is performed. When the CD34-positive cell count according to the first test result is large similar to the myeloblast count according to the second test result, the operator can determine that the validity of the first test result is high, and can validate the first test result. Since the myeloblast count according to the second test result is large and the CD34-positive cell count according to the first test result is large, the operator can determine that the possibility that the subject has hematologic malignancy is high.

When, in the second region 12, a fact that the white blood cell count and the blast cell count are large is displayed in the numerical value display region 521, and there are many images of blast cells in the image display region 522 as well, the operator can grasp a possibility that the subject has AML (acute myeloid leukemia), as in Embodiment 3. In this case as well, when the operator has determined that a first test result necessary for determining contract of AML is not displayed in the first region 11 displayed in contrast, the operator creates a test order for the first test including a necessary antibody reagent, and causes the first test to be performed. With reference to the second test result and the first test result acquired again which are displayed in contrast, the operator can determine whether or not the first test result is valid (e.g., whether or not the first test result reinforces the possibility of contract of AML) as a basis for the possibility of contract of AML obtained in the second test result.

As described above, with reference to the numerical value display region 521 and the image display region 522 included in the second region 12, the operator can grasp a possibility of a predetermined disease. With reference to such a second region 12 and the first region in contrast, the operator can evaluate the validity of the first test result efficiently and in a simple manner in terms of an additional test order as described above.

When a character string indicating the type of blood cell in the numerical value display region 521 is operated by the operator, a blood cell image corresponding to the type of blood cell having been operated is displayed in the image display region 522. Accordingly, the operator can efficiently cause an image that the operator wishes to confirm, out of many blood cell images, to be displayed in the image display region 522. Therefore, evaluation of the validity of the first test result can be efficiently performed.

### <Modification 2 of Embodiment 3>

The second test apparatus 22 may include both of the blood cell counter of Embodiment 3 and the blood cell image analyzer of Modification 1 of Embodiment 3.

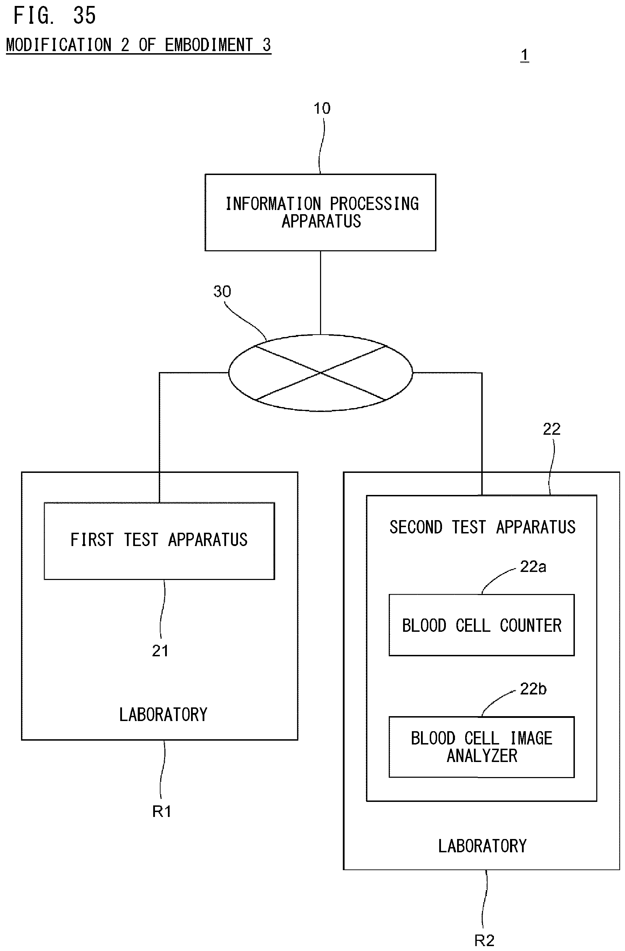

FIG. 35 is a block diagram showing a configuration of the test system 1, according to the present modification.

The second test apparatus 22 is composed of a blood cell counter 22a and a blood cell image analyzer 22b. The blood cell counter 22a is similar to the second test apparatus 22 (blood cell counter) of Embodiment 3 shown in FIG. 30. The blood cell image analyzer 22b is similar to the second test apparatus 22 (blood cell image analyzer) shown in FIG. 33.

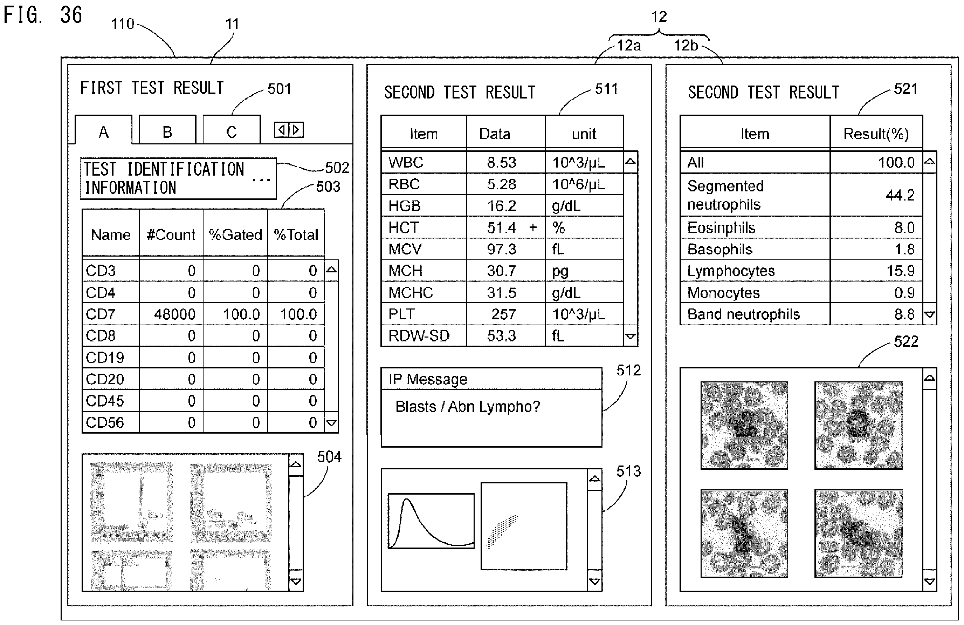

FIG. 36 schematically shows a configuration of the test result screen 110 displayed on the display unit 103 of the information processing apparatus 10, according to the present modification.

When compared with Embodiment 3 shown in FIG. 32, the test result screen 110 of the present modification includes two regions 12a, 12b in the second region 12. The region 12a is similar to the second region 12 of Embodiment 3 shown in FIG. 32, and the region 12b is similar to the second region 12 of Modification 1 of Embodiment 3 shown in FIG. 34. In FIG. 36, the region 11 is disposed adjacent to the region 12a, but the region 11 may be disposed adjacent to the region 12b. For example, from the state in FIG. 36, the region 12a and the region 12b may be disposed so as to be switched with each other in the left-right direction.

In FIG. 36 as well, similar to Modification 1 of Embodiment 3 shown in FIG. 34, when a character string indicating the type of blood cell in the numerical value display region 521 is operated by the operator, a blood cell image corresponding to the type of blood cell having been operated is displayed in the image display region 522. In this case as well, the operator can efficiently cause an image that the operator wishes to confirm, out of many blood cell images, to be displayed in the image display region 522. Therefore, evaluation of the validity of the first test result can be efficiently performed.

In FIG. 36, either one of the regions 12a, 12b may be displayed in accordance with an instruction inputted by the operator through the input unit 104. That is, although both of a second test result by the blood cell counter 22a and a second test result by the blood cell image analyzer 22b are displayed in FIG. 36, either one of the second test results may be displayed in accordance with a switching instruction by a user.

### <Effects of information processing method, information processing apparatus, and test system according to Modification 2 of Embodiment 3>

With the test result screen 110 shown in FIG. 36, with reference to the first test result including the analysis result of surface antigens by the first test apparatus 21, the second test result including the analysis result of blood cells by the blood cell counter 22a, and the second test result including the analysis result of blood cell images by the blood cell image analyzer 22b, the operator can evaluate the validity of the first test result and a possibility of a specific disease. Accordingly, when compared with Embodiment 3 and Modification 1 of Embodiment 3, the efficiency of the test by the first test apparatus 21 (flow cytometer) can further be increased.

Specifically, the operator can evaluate the validity of the first test result with reference to the numerical value display region 503 in the first region 11 corresponding to the first test result and the second region 12 corresponding to the second test results. For example, when a message suggesting the presence of abnormal lymphocytes is displayed in the message display region 512 and an image in which abnormal lymphocytes are present is also displayed in the image display region 522, the values of "CD3", "CD4", "CD19", and "CD20" in the numerical value display region 503 corresponding to the first test result should be, originally, numerical values other than "0". Based on such a background, when the values of "CD3", "CD4", "CD19", and "CD20" in the numerical value display region 503 are "0", the operator can determine that an appropriate test result as the first test result has not been acquired and the first test result displayed in the first region 11 is a test result resulting from failure in preparation of the measurement sample, deterioration of an antibody reagent, or the like. In this case, the operator performs re-preparation of the measurement sample, replacement of the antibody reagent, or the like, and then causes the first test to be performed again, whereby the operator can acquire an appropriate first test result. Here, "CD3", "CD4", "CD19", and "CD20" respectively correspond to a plurality of first test results. Thus, when the operator contrasts each of the plurality of first test results with the second test result, the operator can determine that a retest is necessary, and thus, evaluation of the validity of the first test result can be efficiently performed.

When, in the second region 12, a fact that the white blood cell count is large is displayed in the numerical value display region 511, a message suggesting that many blast cells are present is displayed in the message display region 512, a fact that the white blood cell count and the blast cell count are large is displayed in the numerical value display region 521, and there are many images of blast cells also in the image display region 522, the operator can grasp the highness of the possibility that the subject has AML (acute myeloid leukemia). In this case as well, when the operator has determined that a first test result necessary for determining contract of AML is not displayed in the first region 11 displayed in contrast, the operator creates a test order for the first test including a necessary antibody reagent, and causes the first test to be performed. With reference to the second test result and the first test result acquired again which are displayed in contrast, the operator can determine whether or not the first test result is valid (e.g., whether or not the first test result reinforces the possibility of contract of AML) as a basis for the possibility of contract of AML obtained in the second test result.

### <Modification 3 of Embodiment 3>

In Embodiment 3, the second test apparatus 22 is a blood cell counter, but the second test apparatus 22 may be a genetic test apparatus.

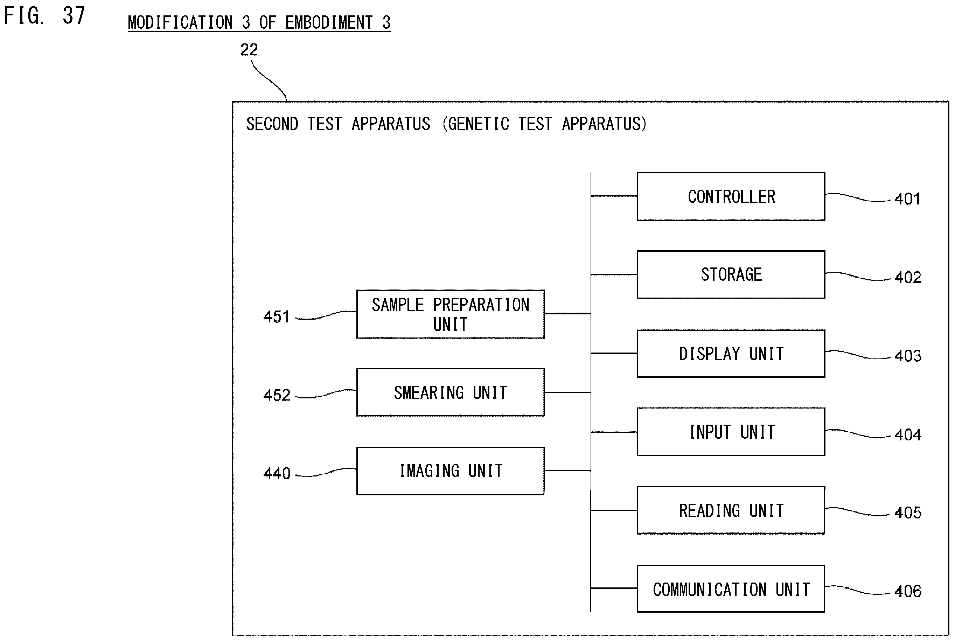

FIG. 37 is a block diagram showing a configuration of the second test apparatus 22 according to the present modification.

The second test apparatus 22 of the present modification is a genetic test apparatus that detects, according to a FISH method, a cell in which an abnormality has occurred in a specific DNA sequence region in the cell, as an abnormal cell. Abnormality in a DNA sequence is gene amplification, deletion, inversion, translocation, or the like. When compared with Modification 1 of Embodiment 3 shown in FIG. 33, the second test apparatus 22 of the present modification includes a sample preparation unit 451 and a smearing unit 452.

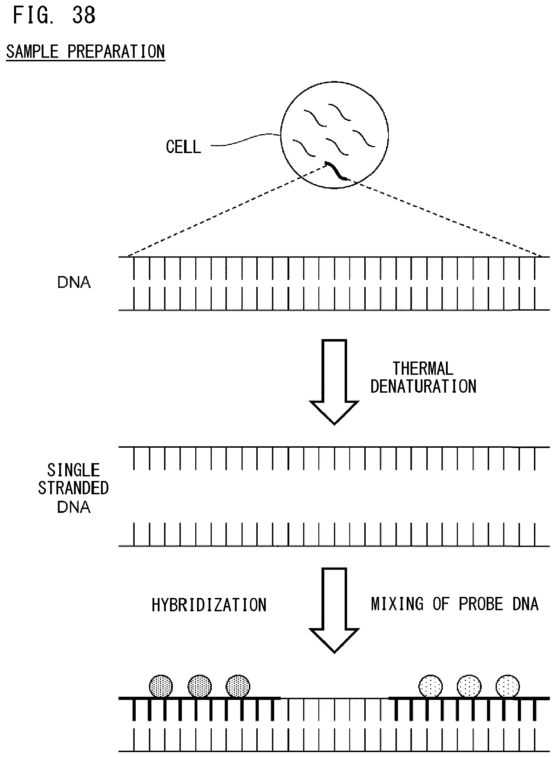

FIG. 38 schematically shows a procedure of sample preparation performed by the second test apparatus 22.

In the present modification, the second specimen is a blood specimen, a specimen collected from a lesion tissue, or the like, and the target cell is a white blood cell, an epithelial cell, or the like, for example.

In the sample preparation unit 451, DNA present in the nucleus of a cell in the second specimen is extracted. In the sample preparation unit 451, the extracted DNA is thermally denatured, whereby the DNA enters a single stranded state. A probe DNA is mixed by the smearing unit 452. The probe DNA includes a fluorescence substance, and binds to the DNA sequence region to be detected. The fluorescence substance included in the probe DNA is configured such that fluorescence is excited by light from the light source 441 (see FIG. 33) of the imaging unit 440. In the sample preparation unit 451, hybridization of the probe DNA is caused. Thus, the DNA sequence region to be detected is labeled by the fluorescence substance, whereby a measurement sample is prepared.

With reference back to FIG. 37, an image of the measurement sample is captured by the imaging unit 440. With reference to FIG. 33, light from the light source 441 is applied to the measurement sample placed on the smear slide 447. An image of fluorescence generated from the fluorescence substance in the measurement sample due to the light from the light source 441 is captured by the camera 446. The controller 401 of the second test apparatus 22 generates a gene image on the basis of image information outputted from the camera 446, detects the state of a predetermined gene on the basis of the generated gene image, and generates a second test result.

For example, when the BCR-ABL fusion gene is to be detected, probe DNAs that respectively bind to the BCR gene and the ABL gene are used. These two probe DNAs include fluorescence substances that emit fluorescences in accordance with lights having wavelengths different from each other. The light source 441 emits two lights having wavelengths different from each other, and the camera 446 receives two fluorescences excited by the respective lights and outputs image information. On the basis of the image information of the two fluorescences, the controller 401 determines that the BCR-ABL fusion gene is present when the positions of the two fluorescences overlap each other. The controller 401 regards the cell including the BCR-ABL fusion gene as a positive cell, and generates a second test result including the number and the ratio of positive cells, and the number and the ratio of negative cells.

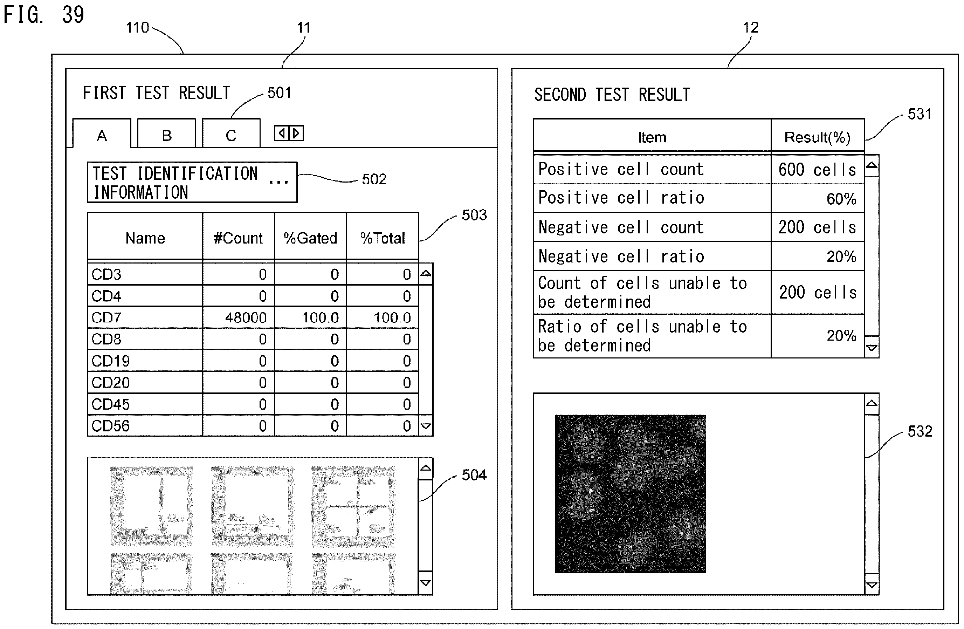

FIG. 39 schematically shows a configuration of the test result screen 110 displayed on the display unit 103 of the information processing apparatus 10, according to the present modification.

When compared with Modification 1 of Embodiment 3 shown in FIG. 34, the test result screen 110 of the present modification is changed in the display in the second region 12. The second region 12 of the present modification includes a numerical value display region 531 and an image display region 532.

In the numerical value display region 531, a positive cell count, a positive cell ratio, a negative cell count, a negative cell ratio, a count of cells unable to be determined, and a ratio of cells unable to be determined, which are based on the captured blood cell image, are displayed. In the image display region 532, the blood cell image captured in the second test, that is, the cell image after FISH staining, is displayed.

### <Effects of information processing method, information processing apparatus, and test system according to Modification 3 of Embodiment 3>

With the test result screen 110 shown in FIG. 39, with reference to the first test result including the analysis result of surface antigens by the first test apparatus 21, and the second test result including the gene analysis result by the second test apparatus 22, the operator can evaluate the validity of the first test result and a possibility of a specific disease. Accordingly, the efficiency of the test by the first test apparatus 21 (flow cytometer) can be increased.

For example, when the BCR-ABL fusion gene is to be detected in the second test, a possibility of chronic myelogenous leukemia (CML) can be determined. Specifically, when the count of white blood cells has been increased, according to the first test result in the first region 11, and the count of positive cells is a predetermined value or larger, according to the second test result in the second region 12, the operator can determine that the validity of the first test result is high, and can validate the first test result. In addition, the operator can determine that the subject has chronic myelogenous leukemia (CML). In this case, the doctor can start a therapy using a molecular target drug targeting the BCR-ABL fusion gene.

The second test apparatus 22 may be a blood coagulation test apparatus. In this case as well, with reference to the first test result and the second test result, the operator can evaluate the validity of the first test result and a possibility of a specific disease (e.g., the type of leukemia). For example, when the count of white blood cells has been increased and the counts of red blood cells and platelets have been decreased, according to the first test result, and D-dimer has a high value and FDP>D-dimer, according to the second test result, the operator can determine that the validity of the first test result is high, and can determine that the subject has AML-M3 (acute myeloid leukemia).

### <Embodiment 4>

In Embodiment 4, an example in which a measurement sample to be measured in the first test apparatus 21 is prepared in a specimen pretreatment device will be described. Unless otherwise specified, the configuration and the process in Embodiment 4 are similar to those in Embodiment 2.

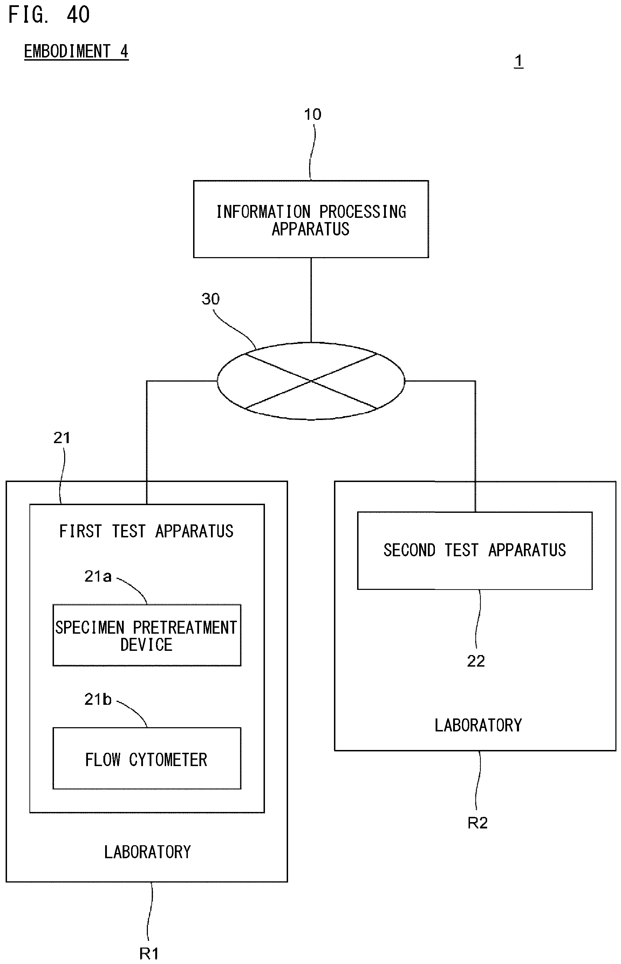

FIG. 40 is a block diagram showing a configuration of the test system 1 according to Embodiment 4.

When compared with Embodiment 1 in FIG. 2, the first test apparatus 21 of Embodiment 4 is composed of a specimen pretreatment device 21a and a flow cytometer 21b. The specimen pretreatment device 21a prepares a measurement sample for the first test from a first specimen. The flow cytometer 21b is similar to the first test apparatus 21 of Embodiment 3 shown in FIG. 28. The information processing apparatus 10 is communicably connected to the specimen pretreatment device 21a and the flow cytometer 21b.

The information processing apparatus 10 transmits an instruction to the specimen pretreatment device 21a so as to prepare a measurement sample from a first specimen, on the basis of a test order for the first test. The specimen pretreatment device 21a prepares a measurement sample from the first specimen on the basis of the test order for the first test. The flow cytometer 21b performs the first test with respect to the measurement sample prepared by the specimen pretreatment device 21a, generates a first test result, and transmits the generated first test result to the information processing apparatus 10. The information processing apparatus 10 displays the first test result and the second test result in parallel to each other in the test result screen 110 as shown in FIGS. 6 to 9.

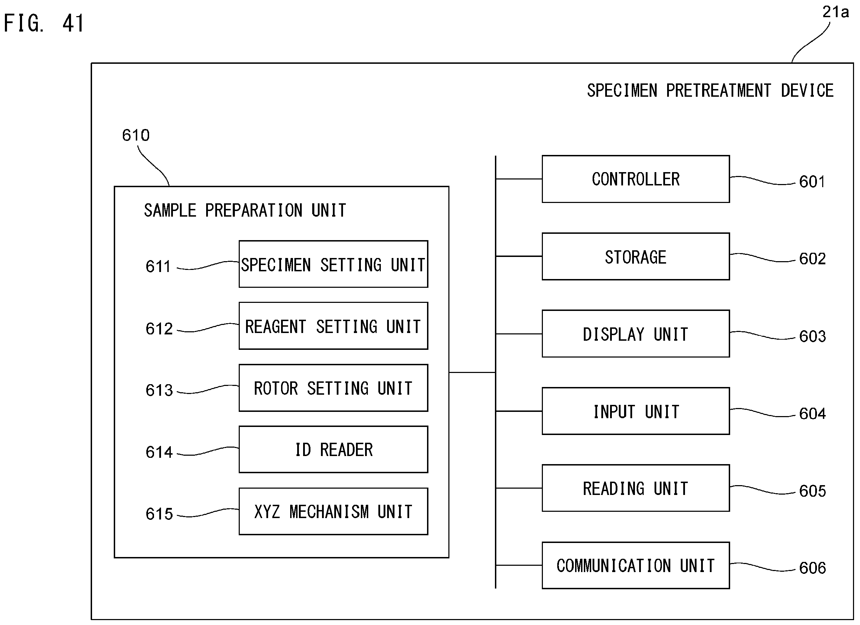

FIG. 41 is a block diagram showing a configuration of the specimen pretreatment device 21a.

The specimen pretreatment device 21a includes a controller 601, a storage 602, a display unit 603, an input unit 604, a reading unit 605, a communication unit 606, and a sample preparation unit 610. The controller 601, the storage 602, the display unit 603, the input unit 604, the reading unit 605, and the communication unit 606 are respectively similar to the controller 301, the storage 302, the display unit 303, the input unit 304, the reading unit 305, and the communication unit 306 of the first test apparatus 21 shown in FIG. 28.

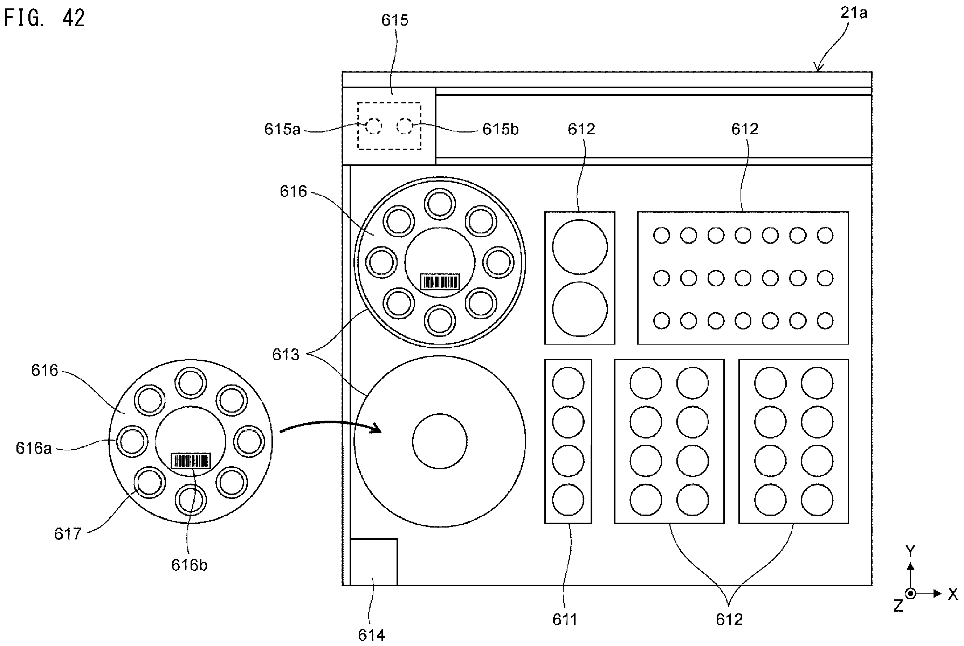

The sample preparation unit 610 includes a specimen setting unit 611, a reagent setting unit 612, a rotor setting unit 613, an ID reader 614, and an XYZ mechanism unit 615. Each component of the sample preparation unit 610 will be described with reference to FIG. 42.

FIG. 42 is a plan view schematically showing a configuration of the specimen pretreatment device 21a. In FIG. 42, X, Y, Z-axes orthogonal to each other are shown for convenience. The Z-axis positive direction is the height direction of the specimen pretreatment device 21a.

In the specimen setting unit 611, a container containing a first specimen is set. A label on which a barcode corresponding to a first specimen ID is printed is attached to this container. The operator reads the barcode of this container by means of the reading unit 605 in advance, inputs, through the input unit 604, at which position in the specimen setting unit 611 the container is to be set, and then, sets the container to the specimen setting unit 611. In the reagent setting unit 612, containers each containing an antibody reagent described with reference to FIGS. 11, 12 are set. Information indicating at which position in the reagent setting unit 612 a container containing which antibody reagent is set is stored in the storage 602 in advance.

In the rotor setting unit 613, a rotor 616 is set. The rotor 616 has a circular shape in a plan view, and in the rotor 616, a plurality of holders 616a each for holding a tube 617 are formed in the circumferential direction. The rotor 616 of the present embodiment includes eight holders 616a, and the eight holders 616a are provided with tube positions of P1 to P8 clockwise. The operator sets an empty tube 617 in a holder 616a in advance, and sets the rotor 616 to the rotor setting unit 613. A label 616b on which a barcode corresponding to a rotor ID for identifying the rotor 616 is attached to the upper face of the rotor 616. The ID reader 614 reads the rotor ID from the label 616b of the rotor 616 set to the rotor setting unit 613.

The XYZ mechanism unit 615 includes a specimen pipette 615a for suctioning and discharging a first specimen, a reagent pipette 615b for suctioning and discharging an antibody reagent, and a mechanism for moving the specimen pipette 615a and the reagent pipette 615b in the X-axis direction, the Y-axis direction, and the Z-axis direction.

When pretreatment with respect to a first specimen is started, the controller 601 drives the XYZ mechanism unit 615 so as to suction the first specimen from the container in the specimen setting unit 611 and dispenses the first specimen into a tube 617 held in the rotor 616, on the basis of an order for the first test described later. The tube 617 is a child specimen container, and the first specimen dispensed in the tube 617 is a child specimen. The controller 601 drives the XYZ mechanism unit 615 so as to suction an antibody reagent from a container in the reagent setting unit 612 and discharge the antibody reagent into the tube 617 held in the rotor 616, on the basis of the order for the first test described later. Accordingly, a measurement sample is prepared in the tube 617.

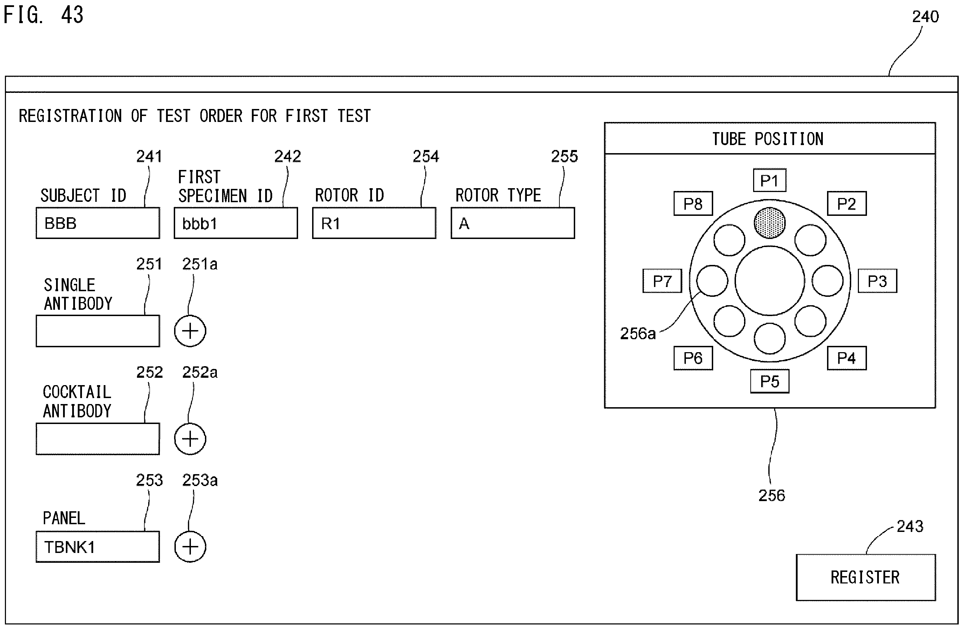

FIG. 43 schematically shows a configuration of the test order registration screen 240 for the first test displayed on the display unit 103 of the information processing apparatus 10, according to Embodiment 4.

When compared with Embodiment 2 shown in FIG. 16, the test order registration screen 240 of Embodiment 4 includes text boxes 254, 255 and a tube position display region 256.

The operator inputs, into the text boxes 254, 255, in which rotor 616 the tube 617 for dispensing the first specimen corresponding to the subject ID and the first specimen ID inputted in the text boxes 241, 242 has been set. In the text box 254, a rotor ID for identifying the rotor 616 is inputted, and in the text box 255, a rotor type for identifying the type of the rotor 616 is inputted.

The tube position display region 256 includes holder images 256a corresponding to the holders 616a, in a schematic image corresponding to the rotor 616. The operator operates a holder image 256a to select at which tube position a measurement sample is to be prepared for each test, with respect to the first specimen corresponding to the subject ID and the first specimen ID. By operating the holder image 256a, the operator can switch the selection state of the tube position between "selected" (with color) and "not selected" (without color).

In the example shown in FIG. 43, with respect to the first specimen corresponding to the subject ID and the first specimen, "TBNK1" is designated as the panel ID, and P1 is selected as the tube position at which a measurement sample is to be prepared. According to the test order in this case, into one tube 617 at the tube position P1, the first specimen and four antibody reagents (see FIG. 12) corresponding to TBNK1 are discharged, whereby a measurement sample is prepared.

When the operator inputs a plurality of single antibody IDs, a plurality of cocktail antibody IDs, and a plurality of panel IDs, the operator operates the addition buttons 251a, 252a, 253a to respectively cause the text boxes 251, 252, 253 to be additionally displayed.

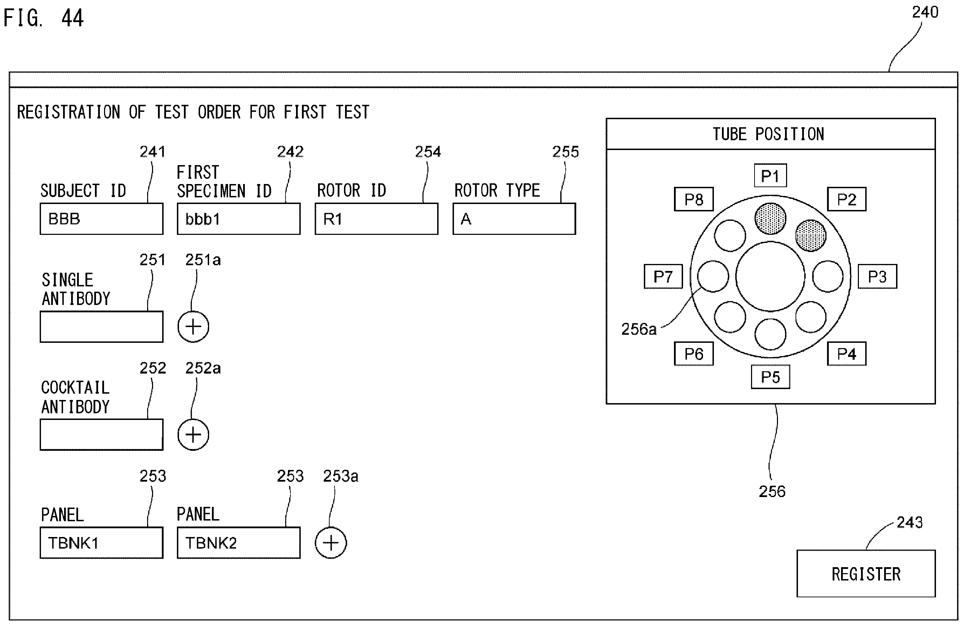

FIG. 44 shows a state where, in the test order registration screen 240 for the first test in FIG. 43, the text box 253 is added through the addition button 253a, and panel IDs "TBNK1" and "TBNK2" are inputted in the two text boxes 253.

In this case, since two measurement samples are prepared so as to correspond to two panels, the operator can select two holder images 256a out of eight holder images 256a. The operator operates holder images 256a to designate two tube positions. According to the test order in this case, one first specimen is dispensed into two tubes 617 at the tube positions P1, P2, four antibody reagents (see FIG. 12) corresponding to TBNK1 are discharged into the tube 617 at the tube position P1, and six antibody reagents (see FIG. 12) corresponding to TBNK2 are discharged into the tube 617 at the tube position P2. Then, in each tube 617, mixing and incubation of the first specimen and the antibody reagents are performed, whereby measurement samples are prepared.

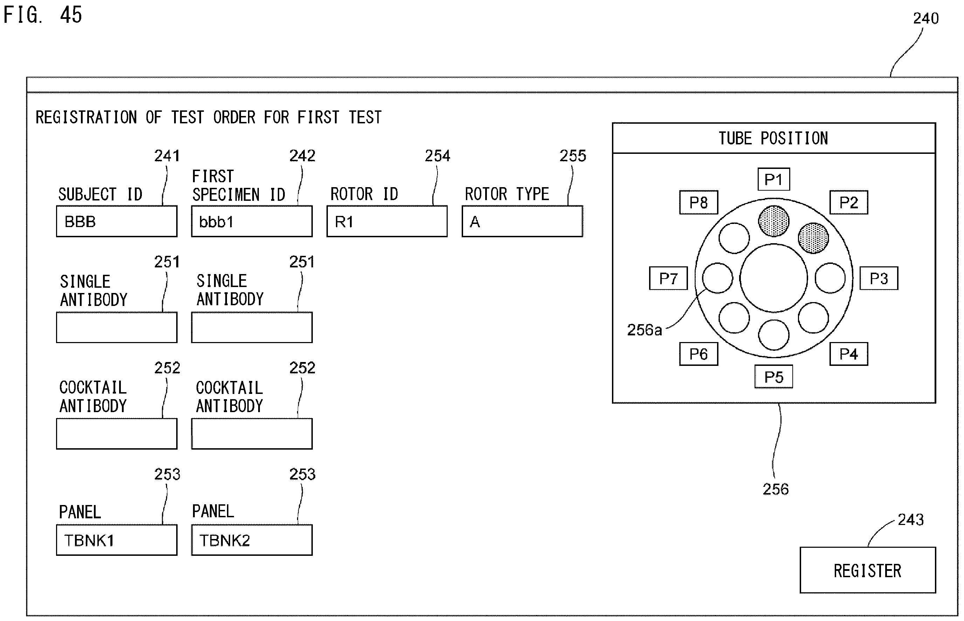

Instead of a text box being added through operation performed on the addition button 251a, 252a, 253a, a necessary text box may be automatically added in accordance with the necessary number of tubes 617 being changed through operation performed on a holder image 256a, as shown in FIG. 45.

In the example shown in FIG. 45, the operator first operates holder images 256a to designate the necessary number of tubes 617 and the tube positions where measurement samples are to be prepared. As shown in FIG. 45, when two holder images 256a enter a selected state, two text boxes 251, 252, 253 are respectively displayed. Then, the operator inputs a single antibody ID, a cocktail antibody ID, or a panel ID in the text boxes 251, 252, 253 such that measurement samples are to be prepared in the two tubes 617.

When the registration button 243 is operated by the operator after the test order for the first test has been designated as shown in FIG. 43 to 45, the controller 101 of the information processing apparatus 10 stores, into the storage 102, the test order for the first test designated in the test order registration screen 240 for the first test.

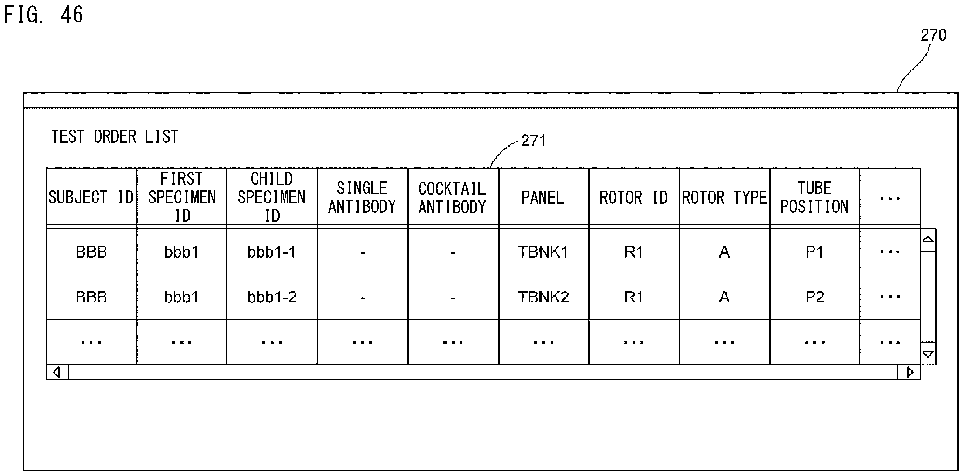

FIG. 46 schematically shows a configuration of the test order display screen 270 for the first test displayed on the display unit 103 of the information processing apparatus 10, according to Embodiment 4.

When compared with Embodiment 2 shown in FIG. 18, items of a rotor ID, a rotor type, and a tube position are added in the list display region 271 of Embodiment 4.

In the first and second rows in the list display region 271, the test order for the first test registered by the registration button 243 being operated in the state shown in FIGS. 44, 45 is shown as an example. In this case, it is understood that, from the identical first specimen shown in the first and second rows, a measurement sample corresponding to TBNK1 is prepared at the tube position P1, and a measurement sample corresponding to TBNK2 is prepared at the tube position P2.

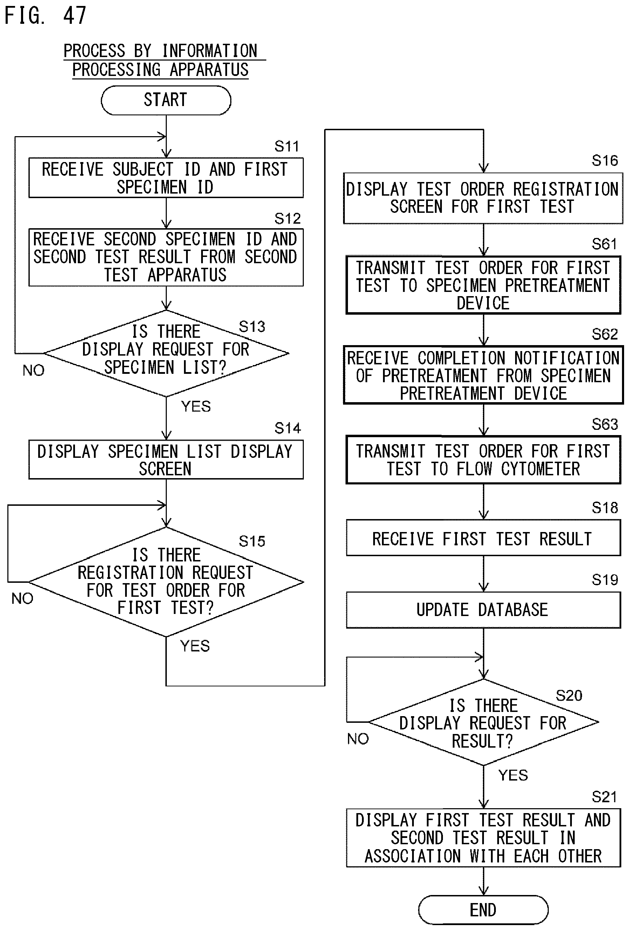

FIG. 47 is a flowchart showing a process performed by the information processing apparatus 10, according to Embodiment 4. In the process in FIG. 47, steps S61 to S63 are added in place of step S17 when compared with FIG. 20. Hereinafter, processes different from those in FIG. 20 will be described.

In step S16, the controller 101 of the information processing apparatus 10 causes the test order registration screen 240 for the first test shown in FIG. 43 to 45 to be displayed. When the registration button 243 in the test order registration screen 240 has been operated, the controller 101 transmits, in step S61, the test order for the first test designated in the test order registration screen 240, to the specimen pretreatment device 21a.

The operator sets a container containing the first specimen serving as a target, to the specimen setting unit 611, and sets the rotor 616 having empty tubes 617 set at designated tube positions, to the rotor setting unit 613. The controller 601 of the specimen pretreatment device 21a controls each component of the specimen pretreatment device 21a such that measurement samples are prepared, on the basis of the test order for the first test received in step S61.

When preparation (pretreatment) of measurement samples has been completed at all tube positions, the controller 101 of the information processing apparatus 10 receives, in step S62, a completion notification of the pretreatment from the specimen pretreatment device 21a. In step S63, the controller 101 transmits the test order for the first test to the flow cytometer 21b. The operator takes out the rotor 616 for which the pretreatment has ended, from the specimen pretreatment device 21a, and sets the rotor 616 to the flow cytometer 21b. The flow cytometer 21b in this case includes a setting unit similar to the rotor setting unit 613, and performs the first test with respect to the measurement sample in a tube 617 for each tube position, on the basis of the test order for the first test. A plurality of first test results obtained from the first test are test results respectively obtained through the first test from the plurality of measurement samples prepared in the tubes 617 different from each other.

The controller 101 receives the first test results from the flow cytometer 21b in step S18, and updates, in step S19, the database 102a shown in FIG. 10, on the basis of the received first test results.

### <Effects of information processing method, information processing apparatus, and test system according to Embodiment 4>

In step S61 in FIG. 47, the controller 101 of the information processing apparatus 10 transmits test identification information including a single antibody ID, a cocktail antibody ID, a panel ID, and the like, to the specimen pretreatment device 21a. Since the specimen pretreatment device 21a and the information processing apparatus 10 are in cooperation with each other in this manner, preparation of measurement samples corresponding to the test content can be automatically performed in the specimen pretreatment device 21a, and the first test can be smoothly performed in the flow cytometer 21b by using the measurement samples automatically prepared in the specimen pretreatment device 21a.

As shown in FIG. 42, the specimen pretreatment device 21a is configured to use the rotor setting unit 613 (setting unit) in which a plurality of tubes 617 (container) for preparing measurement samples are able to be respectively set at the tube positions P1 to P8 (setting position) different from each other, to prepare measurement samples in the tubes 617 (container). As shown in FIG. 46, the information processing apparatus 10 stores the test order for the first test in which the test identification information including a single antibody ID, a cocktail antibody ID, a panel ID, and the like and the tube position (setting position) of a tube 617 (container) are associated with each other. With this configuration, also in a test facility using the specimen pretreatment device 21a that prepares measurement samples by using the rotor setting unit 613 in which a plurality of tubes 617 can be set, the first test by the flow cytometer 21b can be appropriately performed since the test order for the first test in which the test identification information and the tube position are associated with each other is provided to the flow cytometer 21b.

### <Modification of Embodiment 4>

In Embodiment 4, the first test apparatus 21 is composed of the specimen pretreatment device 21a that performs pretreatment (preparation of measurement samples) of the first specimen, and the flow cytometer 21b similar to the first test apparatus 21 of Embodiment 3 shown in FIG. 28. However, not limited thereto, the first test apparatus 21 may be an apparatus in which the specimen pretreatment device 21a and the flow cytometer 21b are integrated.

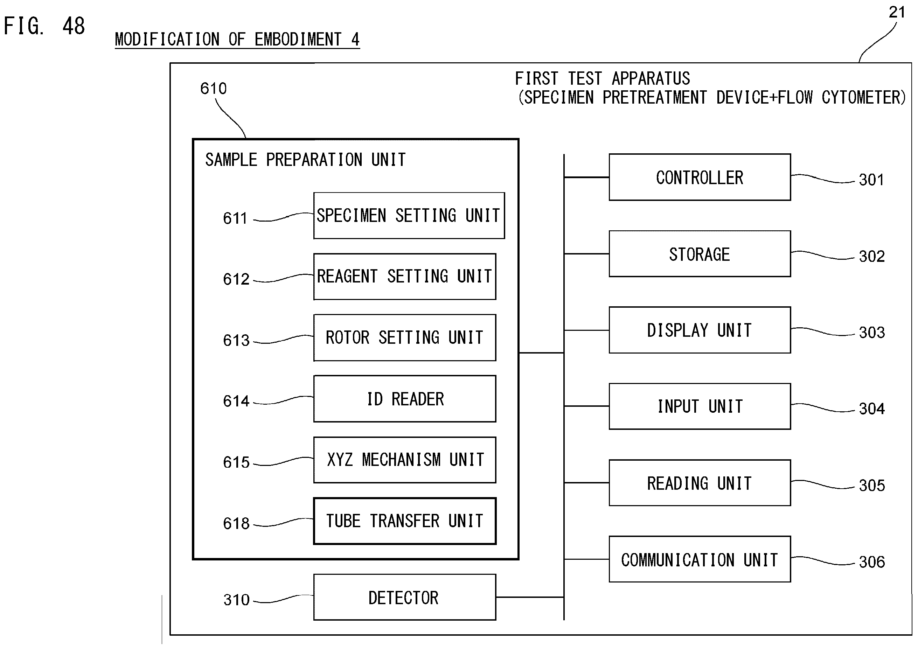

FIG. 48 is a block diagram showing a configuration of the first test apparatus 21 according to the present modification.

In the first test apparatus 21 of the present modification, the sample preparation unit 610 is added when compared with the first test apparatus 21 shown in FIG. 28. In the sample preparation unit 610 of the present modification, a tube transfer unit 618 is added when compared with the sample preparation unit 610 of Embodiment 4 shown in FIG. 41.

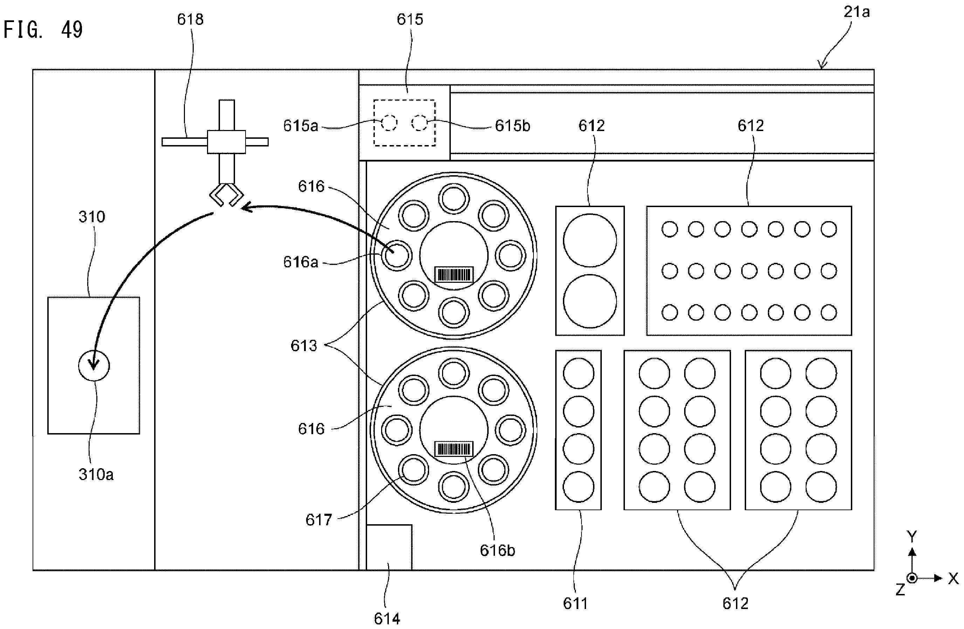

FIG. 49 is a plan view schematically showing a configuration of the first test apparatus 21 according to the present modification.

In the first test apparatus 21 of the present modification, the tube transfer unit 618 and the detector 310 are disposed on the X-axis negative side of the specimen setting unit 611, the reagent setting unit 612, the rotor setting unit 613, the ID reader 614, and the XYZ mechanism unit 615 similar to those of the specimen pretreatment device 21a of Embodiment 4 shown in FIG. 42.

The tube transfer unit 618 transfers a tube 617 containing a measurement sample and held in a holder 616a of the rotor 616, to a holder 310a of the detector 310. The detector 310 suctions the measurement sample in the tube 617 transferred to the holder 310a, and causes the measurement sample to flow in the flow cell 311 as shown in FIG. 29. The detector 310 applies light to each cell in the flow cell 311 from the light sources 312a, 312b, 312c, and light having passed through the cell is detected by the photodetectors 313a, 313b, 313c.

In the present modification, the controller 101 of the information processing apparatus 10 performs a process similar to that in Embodiment 2 shown in FIG. 20. With reference to FIG. 20, when the controller 101 transmits a test order for the first test to the first test apparatus 21 in step S17, the first test apparatus 21 performs pretreatment of preparing measurement samples from the first specimen on the basis of the received test order, and performs the first test with respect to the prepared measurement samples. The controller 101 of the information processing apparatus 10 receives, in step S18, first test results from the first test apparatus 21, and updates, in step S19, the database 102a shown in FIG. 10 on the basis of the received first test results.

Various modifications can be made as appropriate to the embodiments of the present invention, without departing from the scope of the technological idea defined by the claims.

### [Remarks]

Item 1: An information processing method to be executed by a computer, for analysis performed by a flow cytometer capable of acquiring a test result through a test using an antibody reagent, the information processing method comprising:
acquiring a first test result obtained through a first test performed by the flow cytometer with respect to a first specimen of a subject;
acquiring a second test result obtained through a second test different from the first test with respect to a second specimen of the subject; and
displaying the first test result and the second test result in association with each other.

Item 2: The information processing method according to item 1, wherein
the first and second test results are displayed such that the first test result and the second test result are contrastable with each other.

Item 3: The information processing method according to item 1, wherein
the acquiring the first test result includes acquiring the first test result obtained through the first test using the antibody reagent selected in accordance with the second test result.

Item 4: The information processing method according to item 1, further comprising:
displaying a list of information in which information regarding the subject and information regarding the first and second tests are associated with each other; and
receiving a test order for the first test with respect to the subject selected from the list.

Item 5: The information processing method according to item 4, wherein
the acquiring the first test result includes acquiring the first test result with respect to the test order; and
the information processing method further comprises
   associating the information regarding the subject and the first and second test results with each other.

Item 6: The information processing method according to item 4, wherein
the information regarding the subject includes at least one of subject identification information that identifies the subject and specimen identification information that identifies a specimen collected from the subject.

Item 7:The information processing method according to item 1, further comprising:
receiving an input of first specimen identification information that identifies the first specimen; and
receiving second specimen identification information that corresponds to the first specimen identification information and that identifies the second specimen, wherein
the acquiring the first test result includes acquiring the first test result in association with the first specimen identification information, and
the acquiring the second test result includes acquiring the second test result in association with the second specimen identification information.

Item 8: The information processing method according to item 1, further comprising:
displaying the second test result before the acquiring the first test result; and
receiving a test order for the first test after the displaying the second test result.

Item 9: The information processing method according to item 8, wherein
a display screen for the second test result and a reception screen for receiving the test order for the first test are displayed in association with each other.

Item 10:The information processing method according to item 1, wherein
the displaying the first and second test results includes displaying a test result screen including a first region for displaying the first test result and a second region for displaying the second test result.

Item 11:The information processing method according to item 10, wherein
in the second region, an item of a second test result indicating an abnormal value is displayed.

Item 12:The information processing method according to item 10, wherein
a plurality of types of the antibody reagents to be used in the first test are categorized for each container for preparing a measurement sample for the first test, and
the displaying the first test result in the first region includes displaying the first test result for each category of the container in the first region.

Item 13:The information processing method according to item 12, wherein
the displaying the first test result in the first region includes switching and displaying the first test result for each category of the container in the first region in accordance with an operation from a user.

Item 14:The information processing method according to item 1, wherein
the acquiring the first test result includes acquiring the first test result in association with test identification information that identifies the first test.

Item 15:The information processing method according to item 14, wherein
the test identification information includes information indicating the antibody reagent to be used in the first test.

Item 16:The information processing method according to item 14, wherein
the displaying the first and second test results includes displaying the first test result and the test identification information in association with each other.

Item 17:The information processing method according to item 1, wherein
the computer is communicably connected to a specimen pretreatment device configured to prepare a measurement sample for the first test, and
the information processing method further comprises
   transmitting test identification information that identifies the first test, to the specimen pretreatment device.

Item 18:The information processing method according to item 17, wherein
the specimen pretreatment device is configured to use a setting unit in which a plurality of containers for preparing measurement samples are able to be respectively set at setting positions different from each other, to prepare the measurement samples in the containers, and
the information processing method further comprises
   storing a test order in which the test identification information and the setting position are associated with each other.

Item 19:The information processing method according to item 1, wherein
the first test is analysis regarding an antigen present at least at one of a cell surface and inside of a cell, and
the second test is analysis regarding a component different from the antigen.

Item 20:The information processing method according to item 1, wherein
each of the first specimen and the second specimen is a blood specimen.

Item 21:The information processing method according to item 1, wherein
the flow cytometer acquires a plurality of the first test results through the first test using a plurality of types of the antibody reagents.

Item 22:The information processing method according to item 21, wherein
each of the plurality of types of the antibody reagents has a different antibody or a different combination of antibodies.

Item 23:The information processing method according to item 21, wherein
the plurality of the first test results include information on an antigen of a type corresponding to each of the plurality of types of the antibody reagents.

Item 24:The information processing method according to item 21, wherein
the plurality of the first test results are test results respectively obtained through the first test from a plurality of measurement samples prepared in containers different from each other.

Item 25:The information processing method according to item 1, wherein
the second test includes at least one of a test performed by a blood cell counter that uses a flow cell and a test performed by a blood cell image analyzer that captures and analyzes a blood cell image.

Item 26:The information processing method according to item 1, wherein
the second test includes a test performed by a blood cell counter that uses a flow cell and a test performed by a blood cell image analyzer that captures and analyzes a blood cell image, and
the information processing method comprises
   displaying the first test result and the second test result obtained through the second test performed by the blood cell counter and the blood cell image analyzer, in association with each other.

Item 27:The information processing method according to item 1, wherein
the flow cytometer is installed in a laboratory different from a laboratory where the second test is performed.

Item 28:The information processing method according to item 1, wherein
the acquiring the second test result includes receiving the second test result from another computer storing the second test result.

Item 29:An information processing apparatus that is used in analysis performed by a flow cytometer capable of acquiring a test result through a test using an antibody reagent, the information processing apparatus comprising:
a controller; and
a display unit, wherein
the controller is configured to
   acquire a first test result obtained through a first test performed by the flow cytometer with respect to a first specimen of a subject,
   acquire a second test result obtained through a second test different from the first test with respect to a second specimen of the subject, and
   control the display unit to display the first test result and the second test result in association with each other.

Item 30:Atest system comprising:
a flow cytometer capable of acquiring a test result through a test using an antibody reagent;
a test apparatus configured to perform a second test different from a first test performed by the flow cytometer; and
an information processing apparatus connected to the flow cytometer and the test apparatus through a network, wherein
the information processing apparatus includes a controller and a display unit, and
the controller is configured to
   acquire a first test result obtained through a first test performed by the flow cytometer with respect to a first specimen of a subject,
   acquire a second test result obtained through a second test different from the first test with respect to a second specimen of the subject, and
   control the display unit to display the first test result and the second test result in association with each other.

### DESCRIPTION OF THE REFERENCE CHARACTERS

- 1: test system
- 10: information processing apparatus (computer)
- 11: first region
- 12: second region
- 21: first test apparatus (flow cytometer)
- 21a: specimen pretreatment device
- 21b: flow cytometer
- 22: second test apparatus (blood cell counter, blood cell image analyzer, test apparatus)
- 22a: blood cell counter (second test apparatus, test apparatus)
- 22b: blood cell image analyzer (second test apparatus, test apparatus)
- 30: network
- 40: host computer (another computer)
- 101: controller
- 103: display unit
- 110: test result screen
- 231: list display region (list)
- 240: test order registration screen (reception screen)
- 280: display screen
- 613: rotor setting unit (setting unit)
- 617: tube (container)
- P1 to: P8 tube position (setting position)
- R1, R2: laboratory

## Claims

1. An information processing method to be executed by a computer, for analysis performed by a flow cytometer capable of acquiring a test result through a test using an antibody reagent, the information processing method comprising:
acquiring a first test result obtained through a first test performed by the flow cytometer with respect to a first specimen of a subject;
acquiring a second test result obtained through a second test different from the first test with respect to a second specimen of the subject; and
displaying the first test result and the second test result in association with each other.

2. The information processing method according to claim 1, wherein
the first and second test results are displayed such that the first test result and the second test result are contrastable with each other.

3. The information processing method according to claim 1, wherein
the acquiring the first test result includes acquiring the first test result obtained through the first test using the antibody reagent selected in accordance with the second test result.

4. The information processing method according to claim 1, further comprising:
displaying a list of information in which information regarding the subject and information regarding the first and second tests are associated with each other; and
receiving a test order for the first test with respect to the subject selected from the list.

5. The information processing method according to claim 4, wherein
the acquiring the first test result includes acquiring the first test result with respect to the test order; and
the information processing method further comprises
associating the information regarding the subject and the first and second test results with each other.

6. The information processing method according to claim 4, wherein
the information regarding the subject includes at least one of subject identification information that identifies the subject and specimen identification information that identifies a specimen collected from the subject.

7. The information processing method according to claim 1, further comprising:
receiving an input of first specimen identification information that identifies the first specimen; and
receiving second specimen identification information that corresponds to the first specimen identification information and that identifies the second specimen, wherein
the acquiring the first test result includes acquiring the first test result in association with the first specimen identification information, and
the acquiring the second test result includes acquiring the second test result in association with the second specimen identification information.

8. The information processing method according to claim 1, further comprising:
displaying the second test result before the acquiring the first test result; and
receiving a test order for the first test after the displaying the second test result.

9. The information processing method according to claim 8, wherein
a display screen for the second test result and a reception screen for receiving the test order for the first test are displayed in association with each other.

10. The information processing method according to claim 1, wherein
the displaying the first and second test results includes displaying a test result screen including a first region for displaying the first test result and a second region for displaying the second test result.

11. The information processing method according to claim 10, wherein
in the second region, an item of a second test result indicating an abnormal value is displayed.

12. The information processing method according to claim 10, wherein
a plurality of types of the antibody reagents to be used in the first test are categorized for each container for preparing a measurement sample for the first test, and
the displaying the first test result in the first region includes displaying the first test result for each category of the container in the first region.

13. The information processing method according to claim 12, wherein
the displaying the first test result in the first region includes switching and displaying the first test result for each category of the container in the first region in accordance with an operation from a user.

14. An information processing apparatus that is used in analysis performed by a flow cytometer capable of acquiring a test result through a test using an antibody reagent, the information processing apparatus comprising:
a controller; and
a display unit, wherein
the controller is configured to
acquire a first test result obtained through a first test performed by the flow cytometer with respect to a first specimen of a subject,
acquire a second test result obtained through a second test different from the first test with respect to a second specimen of the subject, and
control the display unit to display the first test result and the second test result in association with each other.

15. A test system comprising:
a flow cytometer capable of acquiring a test result through a test using an antibody reagent;
a test apparatus configured to perform a second test different from a first test performed by the flow cytometer; and
an information processing apparatus connected to the flow cytometer and the test apparatus through a network, wherein
the information processing apparatus includes a controller and a display unit, and
the controller is configured to
acquire a first test result obtained through a first test performed by the flow cytometer with respect to a first specimen of a subject,
acquire a second test result obtained through a second test different from the first test with respect to a second specimen of the subject, and
control the display unit to display the first test result and the second test result in association with each other.
